# EUROPEAN PATENT SPECIFICATION

(11) **EP 4 076 291 B1**
(45) Date of publication and mention of the grant of the patent: **15.10.2025**
(21) Application number: 20835883.8
(22) Date of filing: 11.12.2020
(51) Int. Cl.: A61F 2/54, A61F 2/58, A61F 2/68, A61F 2/72, A61F 2/76

(54) **ELECTROMYOGRAPHY AND MOTION BASED CONTROL OF UPPER LIMB PROSTHETICS**
ELEKTROMYOGRAFIE UND BEWEGUNGSBASIERTE STEUERUNG VON PROTHESEN FÜR DIE OBEREN GLIEDMASSEN
COMMANDE DE PROTHÈSE DE MEMBRE SUPÉRIEUR BASÉE SUR L'ÉLECTROMYOGRAPHIE ET LE MOUVEMENT

(30) Priority: 19.12.2019 US 201962950843 P
(43) Date of publication of application: 26.10.2022
(73) Proprietor: Touch Bionics Limited, Livingston Lothian EH54 7EG (GB)
(72) Inventor: BYRNE, Steven, Livingston Lothian EH54 7EG (GB); BENNING, Matthew James, Livingston Lothian EH54 7EG (GB)
(74) Representative: KIPA AB
(86) International application number: PCT/IB2020/061859
(87) International publication number: WO 2021/124060

(56) References cited:
- WO-A2-2014/111843
- US-A1- 2016 287 422
- US-A1- 2018 064 563

## Description

### TECHNICAL FIELD

The present disclosure relates to control of prostheses. More specifically, this disclosure relates to systems, methods, and apparatuses for controlling the operation of an upper limb prosthesis using electromyography (EMG) sensors and motion sensors.

### BACKGROUND

Prostheses are used to replace limbs that amputees have lost and/or are used to provide the function of missing limbs. In particular, efforts have been made to develop prostheses that replace the loss of major limbs, such as legs and arms, in view of the immense impact that such a loss has on the amputee. The loss of upper limbs creates particular challenges due to the intricacy and dexterity of the human hand.

Existing solutions for prosthetics and prosthetic control are limited in their control capability. For instance, existing upper limb prosthetic control systems have limited control input capability resulting in complexity to achieve advanced output control movements. Improvements to these and other drawbacks to controlling prosthetics are desirable.

US 2018/064563A1 discloses features for a prosthetic wrist and associated methods of rotation. The prosthetic wrist attaches to a prosthetic hand. Rotation of the prosthetic wrist is performed in combination with grip formation of the prosthetic hand. An IMU provides data associated with the orientation of the prosthetic hand. A desired grip for the prosthetic hand is selected, for example by gesture control. A rotation for the prosthetic wrist is determined based on the orientation of the prosthetic hand from the IMU data and on the selected desired grip. The prosthetic wrist techniques can also be used in combination with prosthetic arms, elbows and/or shoulders. Example structural configurations for the prosthetic wrist and hand are also provided.

### SUMMARY

The following disclosure describes examples of some embodiments. The invention is defined by the scope of the claims and the examples disclosed herein are present to facilitate the understanding of the systems and methods. For instance, other embodiments of the disclosed systems and methods may or may not include the features described herein. Moreover, disclosed advantages and benefits may apply only to certain embodiments of the invention and should not be used to limit the disclosure. The embodiments disclosed herein each have several aspects no single one of which is solely responsible for the disclosures desirable attributes.

The present disclosure describes systems and methods increasing the functionality and responsiveness of a prosthesis without requiring complex control inputs to achieve the desired movements. A prosthesis may include one or more electromyography sensors and one or more motion sensors, which may achieve increased functionality for a prosthesis as the prosthesis is provided a greater range of control inputs. The prosthesis may detect electromyography (EMG) signals produced by one or more muscles and detect motion signals associated with one or more prosthetics or limbs. The prosthesis may then generate control signals based off of these signals. Advanced analysis techniques may be incorporated into the EMG and motion data control system.

**In** one aspect, the present invention relates to a method of controlling an upper limb prosthetic device as set out in claims 1-7.

Various embodiments of the method aspect, and other aspects, may be implemented. The motion may comprise a motion pattern. The motion may comprise a translation. The motion may comprise a rotation. The muscle contraction may comprise a muscle contraction pattern. The method may further comprise analyzing the EMG data and the at least one of the inertial measurement data or the magnetic field data using a mapping matrix. The method may further comprise entering a control mode for the prosthetic device in response to receiving the EMG data, and then receiving the at least one of the inertial measurement data or the magnetic field data. The method may further comprise monitoring movement of the residual limb to generate a movement threshold, where generating the control signal comprises comparison of the at least one of the inertial measurement data or the magnetic field data with the movement threshold. The method may further comprise replacing the movement threshold with an updated movement threshold, where generating the control signal comprises comparison of the at least one of the inertial measurement data or the magnetic field data with the updated movement threshold. The prosthetic device may comprise one or more of the following: a prosthetic hand, a prosthetic digit, a prosthetic wrist, a prosthetic arm, and a prosthetic elbow, and the control signal may comprise one or more control signals configured to cause one or more of the following: formation of a grip with the prosthetic hand, rotation and/or flexion of the prosthetic digit, rotation of the prosthetic wrist, and rotation of the prosthetic elbow.

In another aspect, the present invention relates to an upper limb prosthetic control system is set out in claims 8-12.

Various embodiments of the prosthetic and/or control system aspects, and of other aspects, may be implemented. The motion may comprise a motion pattern, a translation, or a rotation, and the muscle contraction may comprise a muscle contraction pattern. The processor may be further configured to analyze the EMG data and the at least one of the inertial measurement data and the magnetic field data using a mapping matrix. The processor may be further configured to enter a control mode in response to receiving the EMG data, and then receive the at least one of the inertial measurement data and the magnetic field data. The processor may be further configured to monitor movement of the residual limb to generate a movement threshold, where generating the control signal comprises comparison of the at least one of the inertial measurement data or the magnetic field data with the movement threshold. The prosthetic device may comprise one or more of the following: a prosthetic hand, a prosthetic digit, a prosthetic wrist, a prosthetic arm, and a prosthetic elbow, and the control signal may comprise one or more control signals configured to cause one or more of the following: formation of a grip with the prosthetic hand, rotation of a prosthetic digit, rotation of the prosthetic wrist, and rotation of the prosthetic elbow. In some embodiments, the rotation of the prosthetic digit comprises a closing rotation such as flexion.

In another aspect, the present invention relates to a non-transitory computer-readable medium, having instructions stored thereon that when executed by a processor performs a method of controlling an upper limb prosthetic device, as set out in claims 13-15.

Various embodiments of the non-transitory computer-readable medium aspect, and of other aspects, may be implemented. The motion may comprise a motion pattern, a translation, or a rotation, and the muscle contraction may comprise a muscle contraction pattern. The performed method may further comprise analyzing the EMG data and the inertial measurement data using a mapping matrix. The performed method may further comprise monitoring movement of the residual limb to generate a movement threshold, and where generating the control signal comprises comparison of the inertial measurement data with the movement threshold.

### BRIEF DESCRIPTION OF THE DRAWINGS

The foregoing and other features of the present disclosure will become more fully apparent from the following description and appended claims, taken in conjunction with the accompanying drawings. Understanding that these drawings depict only several embodiments in accordance with the disclosure and are not to be considered limiting of its scope, the disclosure will be described with additional specificity and detail through use of the accompanying drawings. In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the claims. It will be readily understood that the aspects of the present disclosure, as generally described herein, and illustrated in the drawings, may be arranged, substituted, combined, and designed in a wide variety of different configurations, all of which are explicitly contemplated and make part of this disclosure.
Fig. 1A is a perspective view of an embodiment of a partial hand prosthesis having a control system based on electromyography (EMG) and motion sensor data for control of various digits.
Fig. 1B is a perspective view of an embodiment of a hand prosthesis having a control system based on EMG and motion sensor data for control of various digits and/or a wrist.
Fig. 1C is a perspective view of an embodiment of an arm prosthesis having a control system based on EMG and motion sensor data for control of a hand, wrist, and/or elbow.
Fig. 2 is a schematic illustrating an embodiment of a prosthesis designed for transradial amputees including motion and EMG sensors.
Fig. 3 is a schematic illustrating an embodiment of a prosthesis designed for transhumeral amputees including motion and EMG sensors.
Fig. 4 is a block diagram of an embodiment of a control system for controlling a prosthetic by mapping motion data and/or EMG data to a desired prosthesis action.
Fig. 5 is a block diagram of an embodiment of a prosthesis containing a plurality of prosthetic devices and a control system based on motion and EMG data.
Fig. 6 is a data plot illustrating an embodiment of an EMG data profile that may be used in the various control systems and methods described herein.
Fig. 7 is a data plot illustrating an embodiment of an EMG and motion data profile that may be used in the various control systems and methods described herein.
Fig. 8 is a data plot illustrating an embodiment of a dynamic movement threshold for a motion sensor that may be used in the various control systems and methods described herein.
Fig. 9 is a perspective view of an embodiment of a prosthetic hand control profile illustrating various movements that may be detected by a motion sensor that may be used in the various control systems and methods described herein.
Fig. 10 is a flow diagram showing an embodiment of a process for controlling a prosthetic device based on EMG and motion data.
Figs. 11-13 are various front views of a hand prosthetic shown in various configurations after being controlled using the various control systems and methods described herein.

### DETAILED DESCRIPTION

The following detailed description is directed to certain specific embodiments of the development. In this description, reference is made to the drawings wherein like parts or steps may be designated with like numerals throughout for clarity. Reference in this specification to "one embodiment," "an embodiment," or "in some embodiments" means that a particular feature, structure, or characteristic described in connection with the embodiment is included in at least one embodiment of the invention. The appearances of the phrases "one embodiment," "an embodiment," or "in some embodiments" in various places in the specification are not necessarily all referring to the same embodiment, nor are separate or alternative embodiments necessarily mutually exclusive of other embodiments. Moreover, various features are described which may be exhibited by some embodiments and not by others. Similarly, various requirements are described which may be requirements for some embodiments but may not be requirements for other embodiments.

Systems and methods disclosed herein may increase dexterity and functionality of a prosthesis without affecting the ease of use or mobility of the prosthesis. For example, systems and methods disclosed herein may implement one or more EMG sensors in combination with one or more motion sensors. This may provide increased dexterity and functionality to an amputee. In some embodiments, routines and methods disclosed herein may implement EMG sensors placed on a residual limb in combination with a motion sensor placed on either the prosthetic or the residual limb. For instance, the systems and methods may implement two EMG sensors placed on two muscle sites to be used in combination with an inertial measurement sensor (IMS) and/or a magnetic field sensor placed on either the prosthetic or the residual limb. The motion sensor may be an inertial measurement unit (IMU) that includes one or more IMS's. The IMU may include one or more IMS's and a magnetic field sensor.

The resulting EMG data from the EMG sensor and motion data from the one or more motion sensors may be analyzed using various approaches for control of the prosthesis. Such approaches may analyze and/or classify various patterns, clusters, magnitudes, directions, and/or other data-related aspects of the EMG and motion data. Motion thresholds for identifying control inputs may be implemented, which thresholds may be updated. A control signal may be generated based on such analysis or analyses, which may cause one or more prosthetic movements. The prosthetic may be an upper limb prosthetic or component thereof. Such prosthetic movements may thus include digit actuation, hand and/or wrist rotation, and/or elbow rotation. However, the features described herein may be implemented in any prosthesis and is not limited to only upper limb prostheses. Therefore, the prosthesis control approaches described herein could further be implemented, for example, in a lower limb prosthesis such as a foot, ankle, shank, knee, thigh, and/or hip.

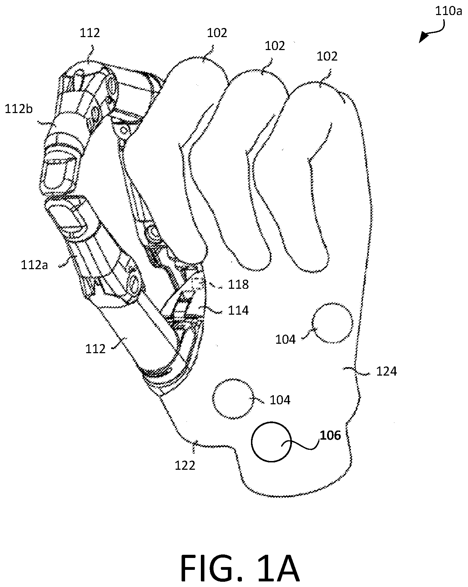

Figs. 1A-1C show example embodiments of various prostheses designed to account for different levels of prosthesis needs, where the prosthesis needs may be based at least in part on the location and type of the residual limb. It will be understood that the prosthesis may include any of the embodiments described herein. It will be further understood that the prosthesis may include any one or more of the following: a prosthetic hand, a prosthetic digit, a prosthetic elbow, a prosthetic wrist, and a prosthetic limb.

With reference to Fig. 1A, a partial hand prosthesis 110a is fitted to a partial-hand amputee that is missing a thumb and forefinger. The prosthesis 110a may be fitted to a residual limb including one or more remaining sound fingers 102 and a sound hand, which may include a thenar muscle group 122 and a hypothenar muscle group 124. The prosthesis 110a comprises two movable digits 112, including a thumb 112a and a forefinger 112b, which are examples of movable components. The digits 112 may then be attached to a body part 114. The body part 114 is then attachable to a residual limb. The digits 112 are arranged such that they may rotate and/or pivot with respect to the body part 114. The digits 112 may be mechanically operated digit members that are moved by an electric motor.

The digits 112 may be a variety of different digits having a variety of different features, such as the digits and/or features described, for example, in U.S. Provisional Application No. 62/935852, filed Nov. 15, 2019, and titled "PROSTHETIC DIGIT ACTUATOR," in U.S. Provisional Application No. 62/902227, filed Sept. 18, 2019, and titled "PROSTHETIC DIGIT ACTUATORS WITH GEAR SHIFTING," in U.S. Provisional Application No. 62/850675, filed May 21, 2019, and titled "ACTUATION SYSTEMS FOR PROSTHETIC DIGITS," in U.S. Provisional Application No. 62/832166, filed April 10, 2019, and titled "PROSTHETIC DIGIT WITH ARTICULATING LINKS," in U.S. Provisional Application No. 62/782830, filed Dec. 20, 2018, and titled "ENERGY CONSERVATION OF A MOTOR-DRIVEN DIGIT," or in U.S. Patent Application No. 16/219556, filed Dec. 13, 2018, and titled "POWERED PROSTHETIC THUMB,". It is however noted, none of said patents and patent applications form part of the scope of protection of the present invention.

The prosthesis 110a and/or residual limb may include one or more EMG sensors 104 and one or more motion sensors 106. As shown, the prosthesis 110A includes two EMG sensors 104 attached to the residual sound hand portion. The motion sensor 106 may be attached to the sound hand or to the prosthesis 110a. The one or more motion sensors 106 may be an inertial measurement sensor (IMS), an inertial measurement unit (IMU) that includes one or more IMS's, an IMU that includes one or more IMS's and a magnetic field sensor, and/or a magnetic field sensor. These features of the sensors 104, 106 may be included in any of the embodiments of the prostheses described herein. Further details of the sensors 104, 106 and related features are described below. The EMG sensors may be any of the EMG sensors and/or include any of the features described, for example, in U.S. Patent No. 9,883,815, titled ELECTROMYOGRAPHY WITH PROSTHETIC OR ORTHOTIC DEVICES, and issued on Feb. 6, 2018. It is however noted, that the subject matter of said patent does not form part of the scope of protection of the present invention.

Fig. 1A illustrates a configuration of the prosthesis 110a operating in "pinch" mode. The prosthesis 110a is operated to bring the thumb 112a and the forefinger 112b into and out of contact with each other. The prosthesis 110a may be configured to make a pinching motion based on one or more control inputs provided to the prosthesis 110a. The configuration illustrated in Fig. 1A may involve direct control of the prosthesis wherein a first control input actuates the thumb 112a and a second control input actuates the forefinger 112b. The thumb 112a and the forefinger 112b may be configured to actuate until the control input is stopped. In some embodiments, the thumb 112a and the forefinger 112b may be configured to actuate until the thumb 112a and the forefinger 112b come into contact with each other. In some embodiments, the configuration may involve a coordinated prepositioning where the thumb 112a and the forefinger 112b are coordinated to move to the predesignated position based on one or more control inputs. In some embodiments, the configuration may involve any combination of coordinated prepositioning and direct control of the thumb 112a and the forefinger 112b. Similar features may be applied to control any other prosthetic digit or digits, such as prosthetic middle, ring, or pinky digits.

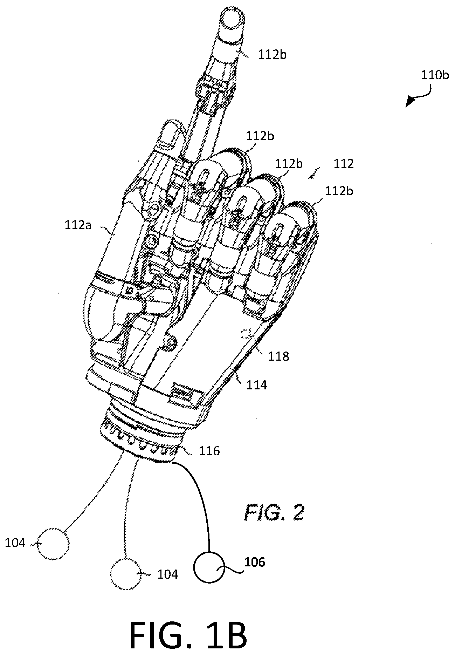

With reference to Fig. 1B, a prosthesis 110b comprises a body part 114 and five digits 112 comprising four fingers 112b and a thumb 112a. The body part 114 is rotatably attached to an attachment component 116, which is used to attach the prosthesis 110b to a wearer. The prosthesis 110b may be attached to a residual limb of the wearer. In this arrangement, the prosthesis 110b is a replacement for the entire hand of the amputee and the residual limb may correspond to an arm, where the prosthesis 110b is configured to attach to the arm of the wearer. In other embodiments, the prosthesis 110b may be configured to attach to other locations on the wearer. In some configurations, the prosthesis 110b may include the prosthesis 110a of Fig. 1A and/or features thereof.

Fig. 1B illustrates a configuration of the prosthesis 110b implementing a "pointing" gesture. The prosthesis 110b is configured to operate the forefinger 112b such that it is extended and to operate the other fingers 112 such that they are closed. The prosthesis 110b may be configured to make the pointing gesture based on one or more control inputs provided to the prosthesis 110b. The configuration illustrated in Fig. 1B may involve direct control of the prosthesis wherein a first control input actuates the forefinger 112b and a second control input actuates the remaining fingers 112b. The fingers 112b may be configured to actuate until the control input is stopped. In some embodiments, the forefinger 112b and the remaining fingers 112b may be configured to actuate until the thumb 112a and the forefinger 112b come into contact with each other. In some embodiments, the configuration may involve a coordinated prepositioning wherein the thumb 112a and the forefinger 112b are coordinated to move to the predesignated position based on one or more control inputs. In some embodiments, the configuration may involve any combination of coordinated prepositioning and direct control of the thumb 112a and the forefinger 112b. The prosthesis 110b may include one or more of the EMG sensors 104 and one or more of the motion sensors 106, as further described herein.

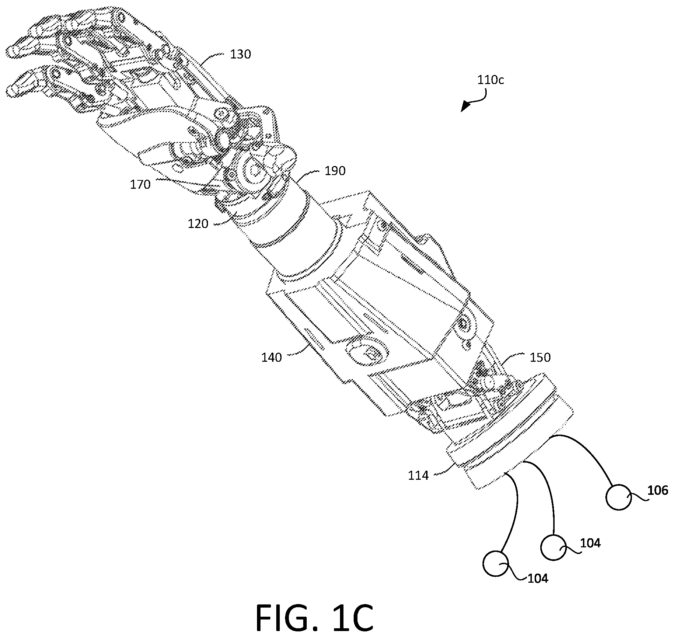

With reference to Fig. 1C, the prosthesis 110c comprises a universal coupler 120, a prosthetic hand 130, a forearm 140, a prosthetic elbow 150, a wrist flexor 170, and a wrist rotator 190. The prosthesis 110c may include any combination of these features. The prosthetic hand 130 may include any number of prosthetic digits, for example as shown in Fig. 1A and Fig. 1B. The prosthesis 110c further includes a body part 114 that connects to the distal end of a user's attachment component, such as a socket. The prosthetic may be attached, via the prosthetic socket, to a residual limb of the wearer. The prosthesis 110c may include one or more of the EMG sensors 104 and one or more of the motion sensors 106, as further described herein.

The prosthetic hand 130, the prosthetic elbow 150, the wrist flexor 170, and the wrist rotator 190 are modular, allowing clinicians to provide an amputee user with only the components they need based on their level of amputation. For example, in the embodiment pictured in Fig. 1C, the prosthesis 110c is a transhumeral prosthesis. In other embodiments, the prosthesis 110c may be a transradial prosthesis and may not include a prosthetic elbow 150. In some configurations, the prosthesis 110c may include any combinations of the prostheses mentioned in Fig. 1A or Fig. 1B. For example, the prosthesis 110c may include the prosthesis 110b indicated in Fig. 1B. In other examples, the prosthesis 110c may include the prosthesis 110a indicated in Fig. 1A. Any combination of the prosthesis 110a and the prosthesis 110b may be considered a modular component of the prosthesis 110c.

Each modular component of the prosthesis 110c may be connected by a controller area network (CAN) bus communication standard for prosthetic arms and a universal coupler 120, allowing users to swap different hands for different applications such as an electric hook, a light weight hand, or a more powerful hand. The universal coupler 120 allows a user to attach, detach, spin, and lock in a component of the arm.

Fig. 1C illustrates a configuration of the prosthesis 110c implementing a gesture, i.e. where the prosthesis 110c is configured to extend the prosthetic hand 130, the prosthetic elbow 150, the wrist flexor 170, and the wrist rotator 190 into a fully extended position. The prosthesis 110c may be configured to make the gesture based on one or more control inputs provided to the prosthesis 110c. The configuration illustrated in Fig. 1C may involve control, for example direct control, of the prosthesis wherein a first control input actuates one or more of the prosthetic hand 130, the prosthetic elbow 150, the wrist flexor 170, and the wrist rotator 190 and a second control input actuates a different one of, or a different configuration of, one or more of the prosthetic hand 130, the prosthetic elbow 150, the wrist flexor 170, and the wrist rotator 190. Actuation of the prosthetic hand 130 may include actuation of one or more of the digits thereon. The one or more of the prosthetic hand 130, the prosthetic elbow 150, the wrist flexor 170, and the wrist rotator 190 may be configured to actuate until the control input is stopped. In some configurations, the prosthetic hand 130, the prosthetic elbow 150, the wrist flexor 170, and the wrist rotator 190 may be configured to actuate until a fully extended position is reached.

In some embodiments, the configuration may involve a coordinated prepositioning. For example, the prosthetic hand 130, the prosthetic elbow 150, the wrist flexor 170, and/or the wrist rotator 190 may be coordinated to move to a predesignated position or configuration based on one or more control inputs. In some embodiments, the configuration may involve any combination of coordinated prepositioning and direct control of one or more of the prosthetic hand 130, the prosthetic elbow 150, the wrist flexor 170, and the wrist rotator 190.

Each of the digits 112, the prosthetic hand 130, the prosthetic elbow 150, the wrist flexor 170, and the wrist rotator 190 as seen in Figs. 1A, 1B, and 1C may be actuated. For example, these features may pivot with respect to the body part 114 and flex and extend to mimic a sound human body part. In addition to flexing and extending, the thumb digit 112a and the wrist rotator 190 may also pivot, for example rotate with the hand 130, with respect to the body part 114. The body part 114 may also be a movable component. For example, in the case of the full-hand prosthesis as seen in Fig. 1B the body part 114 may rotate relative to the attached component 116, which is fitted to the wearer of the prosthesis. The body part 114 may be motor driven with respect to the attachment component 116. In this case, the body part 114 may perform the function of wrist rotation in the same manner as a human hand.

The configurations and gestures illustrated in Figs. 1A, 1B, and 1C may be considered to be example operating modes of the digits 112, the prosthetic hand 130, the prosthetic elbow 150, the wrist flexor 170, and the wrist rotator 190 of the prostheses 110a, 110b, and 110c. The rotation of the body part 114, for example relative to the attached component 116 or other components, may also be considered as an operating mode of the body part 114 of the prostheses 110a, 110b, and 110c. The direction of rotation of the body part 114 may also be considered as an operating mode. The prostheses 110a, 110b, and 110c may thus be considered as having a plurality of operating modes. The operating modes are selected by the wearer of the prostheses 110a, 110b, and 110c depending on the operation they wish the prostheses 110a, 110b, and 110c to perform.

One or more of the digits 112, the prosthetic hand 130, the prosthetic elbow 150, the wrist flexor 170, and the wrist rotator 190 may also have a number of operating conditions. One or more of the digits 112, the body part 114, the prosthetic hand 130, the prosthetic elbow 150, the wrist flexor 170, and/or the wrist rotator 190 may have a number of operating conditions, including but not limited to any combination of the following: direction of movement, speed of movement, acceleration, deceleration, applied force, operating duration, amount of extension, amount of flexion, and angle of rotation. The prostheses 110a, 110b, and 110c may thus be considered as having a plurality of operating conditions. The operating conditions may be selected by the wearer of the prostheses 110a, 110b, and 110c depending on the operation they wish the prostheses 110a, 110b, and 110c to perform, as further described herein.

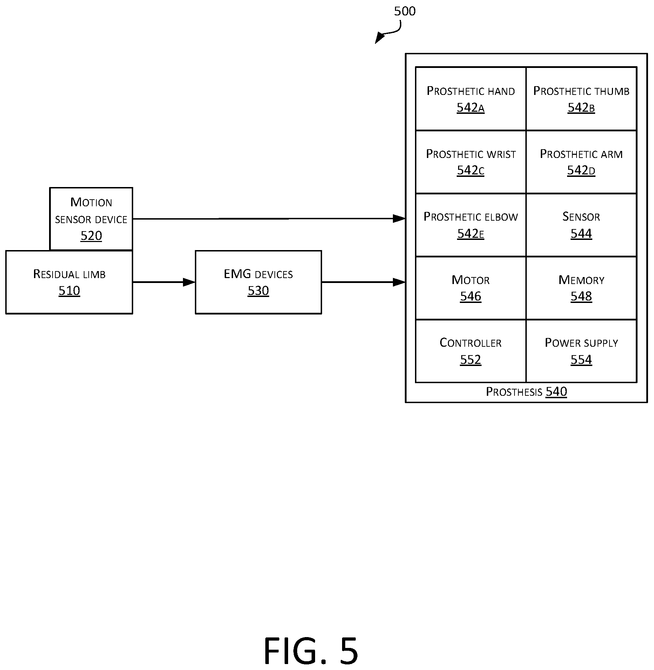

The prostheses 110a, 110b, and 110c also comprise a controller, as further described herein for example with respect to Fig. 5, which controls operations of one or more of the digits 112, the prosthetic hand 130, the prosthetic elbow 150, the wrist flexor 170, and the wrist rotator 190. The controller may include a processor and firmware which together control the operation of the one or more of the digits 112, the prosthetic hand 130, the prosthetic elbow 150, the wrist flexor 170, and the wrist rotator 190.

The prostheses 110a, 110b, and 110c may receive data from the EMG sensor 104 and/or the motion sensor 106. For example, the prostheses may receive EMG data from one, two, or more electromyographic (EMG) sensors 104, located, for example, on the residual limb of the wearer and that detect EMG signals from the user's residual body. Each prosthesis 110a, 110b, and 110c may include any number of EMG sensors 104. As shown in Fig. 1A, the partial-hand prosthesis 110a includes two EMG sensors 104 located on the thenar muscle group 122 and the hypothenar muscle group 124 of the residual limb. As shown in Fig. 1B, the full-hand prosthesis 110b includes the two EMG sensors 104 configured to be located on, for example, the muscle groups of the arm of the wearer. As shown in Fig. 1C, the partial-arm prosthesis 110c includes the two EMG sensors 104 configured to be located on, for example, the muscle groups of the residual limb of the wearer. In some embodiments, the two EMG sensors 104 may be located on other areas that provide electrophysiological signals for the sensors. For example, the two EMG sensors 104 may be located elsewhere on the body of the wearer outside of the residual limb of the wearer. The electrophysiological signals produced from the muscles to which the EMG sensors 104 are attached may be proportional to the activity of the muscles.

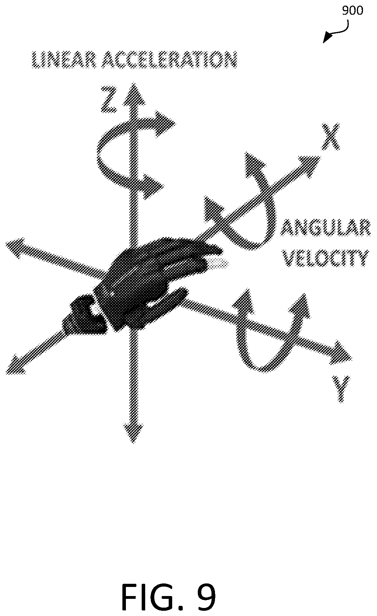

The prostheses 110a, 110b, and 110c may receive motion data from the one or more motion sensors 106. The motion sensor 106 may be located on the residual limb of the wearer and/or on the prosthesis. One or more of the prostheses 110a, 110b, and 110c may include any number of the motion sensors 106. In some embodiments, the motion sensors 106 may be located on the prosthesis 110a, 110b, and/or 110c. In some embodiments, at least a first motion sensor 106 may be located on the prosthesis 110a, 110b, and/or 110c and at least a second motion sensor 106 may be located on the residual limb of the wearer. In some embodiments, the motion sensor 106 may be located at any location on the wearer. As shown in Fig. 1A, the partial-hand prosthesis 110a includes the motion sensor 106 located on the sound hand of the wearer. As shown in Fig. 1B, the full-hand prosthesis 110b includes the motion sensor 106 configured to be located on, for example, any location on the arm of the wearer. As shown in Fig. 1C, the partial-arm prosthesis 110c includes the motion sensor 106 configured to be located on, for example, any location on the residual limb of the wearer. In some embodiments, the motion sensor 106 may be located on other areas of the user that provides a motion signal. The motion sensor signals produced from the apparatus to which the motion sensor is attached may be proportional to the motion of the residual limb. The motions may be any of the motions, such as translations and/or rotations, as further described herein, for example with respect to FIG. 9.

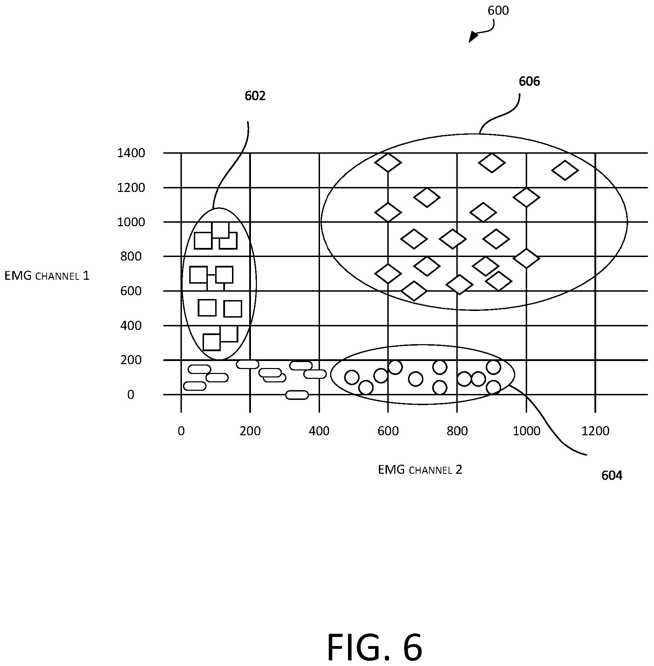

The EMG data and/or motion data may be used to generate input control signals for control of the prostheses 110a, 110b, and/or 110c, including the digits 112, the prosthetic hand 130, the prosthetic elbow 150, the wrist flexor 170, and/or the wrist rotator 190. The EMG and motion data may be used to perform an action or achieve a desired configuration. In some embodiments, the EMG and motion data may be used for proportional control of the one or more prostheses. For example, the wearer may proportionally control the speed of the operation of the digits 112. Any number of EMG sensors 104 and motion sensors 106 could be used to control the operation of the prostheses 110a, 110b, and 110c. As further described herein, for example with respect to Figs. 6-7, the motion sensor data and/or the EMG data may correspond to a matrix mapping that generates control signals to result in a predetermined movement by the one or more prostheses.

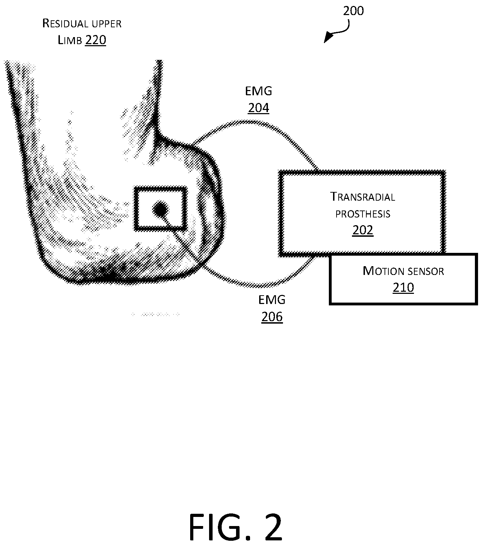

Fig. 2 is a schematic illustrating an embodiment of a system 200 having a prosthesis 202 designed for transradial amputees and including motion and EMG sensors. Fig. 2 shows an example implementation of the system 200 and the prosthesis 202. The system 200 includes the prosthesis 202 as a transradial prosthesis. The system 200 may include a residual upper limb 220, a first EMG sensor 204, a second EMG sensor 206, a motion sensor 210, and the transradial prosthesis 202.

The residual upper limb 220 may include the lower portion of the upper limb, i.e. below the elbow, to which the transradial prosthesis 202 is configured to attach. The residual upper limb 220 may include a socket configured to attach to the transradial prosthesis 202. In some implementations, the socket may be configured to attach to both the residual upper limb 220 and the transradial prosthesis 202. The transradial prosthesis 202 may include any of the prostheses described herein. For example, the transradial prosthesis 202 may include an elbow prosthesis, an elbow joint, a lower arm prosthesis, a wrist prosthesis, a hand prosthesis, and/or prosthetic digits.

The first EMG sensor 204 and the second EMG sensor 206 may be attached to the residual upper limb 220. In another implementation, the first EMG sensor 204 and the second EMG sensor 206 may be attached to any location on the body of the wearer. The first EMG sensor 204 may detect a first electrophysiological signal and the second EMG sensor 206 may detect a second electrophysiological signal. The first electrophysiological signal and the second electrophysiological signal may correspond to one or more muscle contractions. The first EMG sensor 204 and the second EMG sensor 206 may provide both the first and the second electrophysiological signals to the transradial prosthesis 202. In other implementations, the system 200 may include more than two EMG sensors. In some implementations, the system 200 may include any number of EMG sensors that receive any number of electrophysiological signals that are then provided to the transradial prosthesis 202. In some implementations, the EMG sensors are provided in pairs corresponding to muscle groups. In other implementations, the EMG sensors are provided in isolation.

The motion sensor 210, in this configuration, is attached to the transradial prosthesis 202. In other implementations, the motion sensor 210 may be attached to the residual upper limb 220 of the wearer. In another implementation, the motion sensor 210 may be attached to any location of the wearer. The motion sensor 210 may detect any motion of the location where the motion sensor is placed. The motion sensor 210 may include any combination of accelerometers, gyroscopes, magnetometers, and other devices configured to detect motion. In some embodiments, the motion sensor 210 may be an inertial measurement unit (IMU) that includes one or more inertial measurement sensors (IMS). In some embodiments, the motion sensor 210 may be an IMS.

The IMS may provide inertial measurement data relating to movement characteristics of the IMS and thus of the prosthesis or residual limb. The IMS may provide motion data related to the speed, direction, angle, etc. of the prosthesis or residual limb, or any of the operating conditions as further described herein, for example with respect to Figs. 1A-1C.

The motion sensor 210 may include or be a magnetic field sensor. In some embodiments, the motion sensor 210 may be an IMU that includes one or more IMS's and one or more magnetic field sensors. The magnetic field sensor may provide magnetic field data corresponding to the orientation of the magnetic field sensor and thus of the corresponding prosthesis or residual limb to which it is attached. The motion sensor 210 may provide the motion data to the transradial prosthesis 202, for example a controller thereof. In some implementations, the system 200 may include more than one motion sensor 210. In some implementations, the system 200 may include any number of motion sensors 210 that detects any number of motion signals for which related motion data is provided to the controller of the transradial prosthesis 202.

The transradial prosthesis 202, such as a controller thereof, may receive the EMG and motion data. The transradial prosthesis 202 may receive at least the first electrophysiological data corresponding to a first EMG sensor 204, the second electrophysiological data corresponding to a second EMG sensor 206, and the motion sensor data corresponding to the motion sensor 210. The transradial prosthesis 202 may be attached to the residual upper limb 220, where the wearer of the transradial prosthesis 202 has retained motion of both the shoulder joint and the elbow joint. The wearer may provide fluid linear and angular movement of the residual upper limb 220 in all axes, except for angular movement about the X-axis. In other embodiments, the wearer may provide movement of the residual upper limb 220 that includes movement in the X-axis. In certain embodiments, the transradial prosthesis 202 may include any combination of the following modular components: powered digits, a powered hand, a wrist flexor, and a wrist rotator. The transradial prosthesis 202 may include other modular components in other implementations. In some implementations, the transradial prosthesis 202 may include modular components that are not powered.

The various prostheses described herein may be controlled according to a mapping matrix. Table 1, shown below, provides an example prosthesis function mapping matrix that may be used with the various prosthesis systems described herein, such as for the wearer of the transradial prosthesis 202. Table 1 lists user inputs (first two columns) and the corresponding prosthesis output (last two columns). User inputs may include EMG inputs (first column), shown here as muscle contractions, and/or motion inputs (second column), shown here as residual limb inputs. The prosthesis outputs may include direct control (third column) and/or prepositioning (fourth column). A given row provides a set of user input(s) and the corresponding prosthesis output(s). For example, the first row shows that a resting muscle (no contraction) with no detected residual-limb motion may result in no control outputs. The second row shows that a muscle contraction detected as Pattern 1 and with no residual limb motion may result in opening a prosthetic hand, etc.

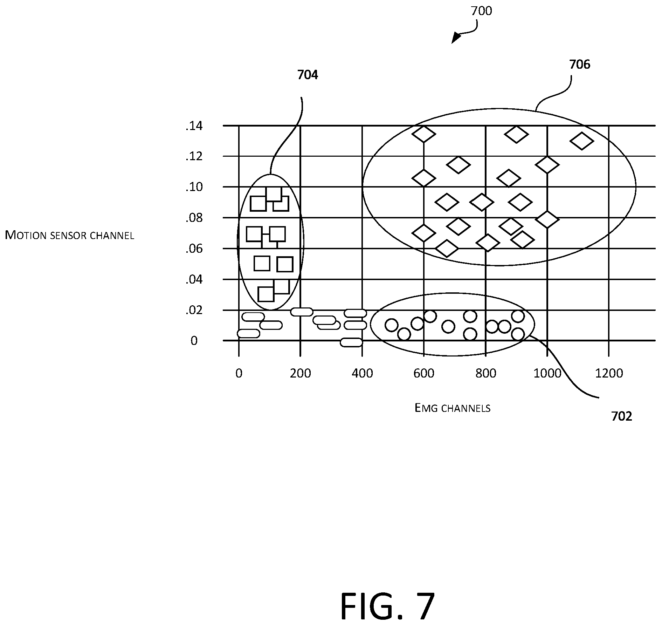

In some embodiments, pattern 1 corresponds to the activation of the first EMG sensor, pattern 2 corresponds to the activation of the second EMG sensor, and pattern 3 corresponds to activation of the two EMG sensors and the motion sensor. As seen in and further explained in Fig. 7, the patterns 1, 2, and 3 may correspond to the different inputs provided to the transradial prosthesis 202. In the example shown in Table 1, the transradial prosthesis 202 includes two myoelectric prosthetic devices: a powered wrist rotator, and a powered hand. The individual activation of the first EMG sensor 204 and the second EMG sensor 206 is mapped to controlling the opening and closing functions of the prosthetic hand, respectively. In Table 1, the angular residual limb movements are mapped to controlling the wrist rotator device and the powered rotating thumb while the linear residual limb movements are mapped to controlling the coordinated prepositioning functions. This is merely an example mapping scheme. In other embodiments, the muscle contraction patterns may be mapped to the control of different modular components, including modular components that are not depicted in the current embodiment.

**TABLE 1**

| **USER INPUT** | | **PROSTHESIS OUTPUT** | |
|---|---|---|---|
| MUSCLE CONTRACTION | RESIDUAL-LIMB MOTION | DIRECT CONTROL | PREPOSITIONING |
| REST | - | NONE | - |
| PATTERN 1 | - | HAND OPEN | - |
| PATTERN 2 | - | HAND CLOSE | - |
| PATTERN 3 | NO MOTION | NONE | - |
| PATTERN 3 | ANGULAR MOTION *(LEFT)* | WRIST ROTATE *(COUNTERCLOCKWISE)* | - |
| PATTERN 3 | ANGULAR MOTION *(RIGHT)* | WRIST ROTATE *(CLOCKWISE)* | - |
| PATTERN 3 | ANGULAR MOTION *(DOWN)* | THUMB ROTATE *(PALMAR)* | - |
| PATTERN 3 | ANGULAR MOTION *(UP)* | THUMB ROTATE *(LATERAL)* | - |
| PATTERN 3 | LINEAR MOTION *(LEFT)* | - | LATERAL GRIP |
| PATTERN 3 | LINEAR MOTION *(RIGHT)* | - | PINCH GRIP |
| PATTERN 3 | LINEAR MOTION *(FORWARD)* | - | INDEX POINT |
| PATTERN 3 | LINEAR MOTION *(BACKWARD)* | - | TRIPOD GRIP |

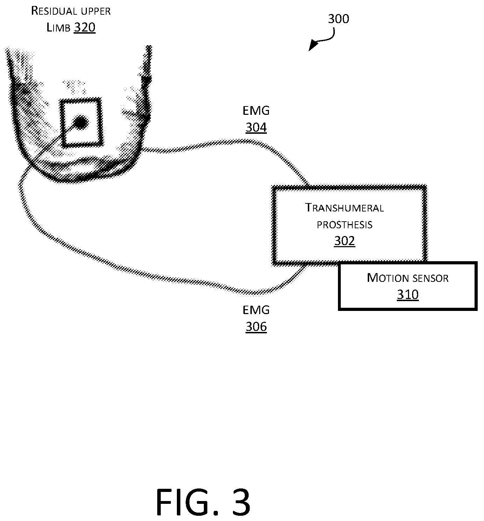

Fig. 3 represents another example implementation of a prosthesis. Fig. 3 represents a system 300 providing an example implementation of a transhumeral prosthesis. The system 300 may include a residual upper limb 320, a first EMG sensor 304, a second EMG sensor 306, a motion sensor 310, and a transhumeral prosthesis 302. The system 300 may include any of the features and/or functions as described with respect to the system 200, and vice versa.

The residual upper limb 320 may include the upper portion of an upper limb or any limb to which the transhumeral prosthesis 302 may be attached. The residual upper limb 320 may be configured to attach directly or indirectly to the transhumeral prosthesis 302. In some embodiments, a first device may be configured to attach to both the residual upper limb 320 and the transhumeral prosthesis 302. For example, a second prosthesis may be configured to attach to the residual upper limb 320 and the transhumeral prosthesis 302 may be configured to attach to the second prosthesis. In another example, a spacer may be configured to attach to the residual upper limb 320 and the transhumeral prosthesis 302.

The first EMG sensor 304 and the second EMG sensor 306 may be attached to the residual upper limb 320. In another embodiment, the first EMG sensor 304 and the second EMG sensor 236 may be attached to any location on the body of the wearer. The first EMG sensor 304 may detect a first electrophysiological signal and the second EMG sensor 306 may detect a second electrophysiological signal. The first EMG sensor 304 and the second EMG sensor 306 may provide both the first and the second electrophysiological signals to the transhumeral prosthesis 302, such as a controller thereof. In other embodiments, the system 300 may include more than two EMG sensors. In some embodiments, the system 300 may include any number of EMG sensors that receive any number of electrophysiological signals that are then provided to the transhumeral prosthesis 302. In some implementations, the EMG sensors are provided in pairs corresponding to muscle groups. In other implementations, the EMG sensors are provided in isolation.

The motion sensor 310, in this configuration, is attached to the transhumeral prosthesis 302. In some embodiments, the motion sensor 310 may be attached to the residual upper limb 320 of the wearer. In some embodiments, the motion sensor 310 may be attached to any location of the wearer. The motion sensor 310 may detect any motion of the location where the motion sensor is placed. The motion sensor 310 may include any combination of accelerometers, gyroscopes, magnetometers, and other devices configured to detect motion. In some implementations, the motion sensor 310 may be an inertial measurement sensor. The inertial measurement sensor may provide inertial measurement data corresponding to the location of the inertial measurement sensor. In other implementations, the motion sensor 310 may be a magnetic field sensor. The magnetic field sensor may provide magnetic field data corresponding to the location of the magnetic field sensor. The motion sensor 310 may provide the motion signal to the transhumeral prosthesis 302. In some implementations, the system 300 may include more than one motion sensor. In some implementations, the system 300 may include any number of motion sensors that receive any number of motion signals that are then provided to the transhumeral prosthesis 302.

The transhumeral prosthesis 302 may receive at least the first electrophysiological signal, the second electrophysiological signal, and the motion sensor signal. The transhumeral prosthesis 302 may be attached to a residual upper limb 320 of the wearer, where the wearer has retained motion of the shoulder joint. The wearer may provide fluid angular movement of the residual upper limb 320 in one or more directions, such as in the x and/or y axes, where the z axis extends along the direction of extension of the upper portion of the upper limb. In some embodiments, the wearer may provide rotation of the residual upper limb 320 about any of these axes and/or the wearer may provide linear movement of the residual upper limb 320. In certain embodiments, the transhumeral prosthesis 302 may include any combination of the modular components: powered digits, a powered elbow, a powered wrist rotator, a powered wrist flexor, and a multi-articulating hand. The transhumeral prosthesis 302 may include other modular components. In some embodiments, the transhumeral prosthesis 302 may include modular components that are not powered.

The various prostheses described herein may be controlled according to a mapping matrix. Table 2, shown below, provides an example prosthesis function mapping matrix, which may be used for the wearer to control the transradial prosthesis 302. As described above with respect to Table 1, a given row corresponds to a set of user input(s) and prosthesis output(s) For example, pattern 1 may correspond to the activation of the first EMG sensor, pattern 2 may correspond to the activation of the second EMG sensor, and pattern 3 may correspond to activation of the two EMG sensors and the motion sensor. As seen in and further explained in Fig. 7, the patterns 1, 2, and 3 may correspond to the different inputs provided to the transhumeral prosthesis 302. For the example control scheme shown in Table 2, the transhumeral prosthesis 302 may include three myoelectric prosthetic devices: a powered wrist rotator, a powered elbow, and a powered hand. In Table 2, the individual activation of the first EMG sensor 304 (pattern 1) and the second EMG sensor 306 (pattern 2) is mapped to controlling the opening and closing functions of the prosthetic hand, respectively. In Table 2, the powered elbow and the wrist rotator devices are actuated by the combination of a distinct EMG signal, shown as a muscle contraction, and a specific motion signal, shown as a residual upper limb movement.

In some embodiments, the muscle contraction patterns may be mapped to the control of different modular components, including modular components and/or movements that are not explicitly depicted in the embodiments described herein. The mapping of the residual upper limb movements to prosthesis functions may be chosen such as to mimic natural movement and/or be customized to a particular user's preferences. The transradial prosthesis 202 and the transhumeral prosthesis 302 may allow control of multiple prosthetic devices without the need for mode switching or muscle-pulsing alone due to the additional control inputs available via the residual upper limb movement.

**TABLE 2**

| **USER INPUT** | | **PROSTHESIS OUTPUT** | |
|---|---|---|---|
| MUSCLE CONTRACTION | RESIDUAL-LIMB MOTION | DIRECT CONTROL | PREPOSITIONING |
| REST | - | NONE | - |
| PATTERN 1 | - | HAND OPEN | - |
| PATTERN 2 | - | HAND CLOSE | - |
| PATTERN 3 | NO MOTION | NONE | - |
| PATTERN 3 | ANGULAR MOTION *(SHOULDER EXTENSION)* | ELBOW FLEXION | - |
| PATTERN 3 | ANGULAR MOTION *(SHOULDER FLEXION)* | ELBOW EXTENSION | - |
| PATTERN 3 | ANGULAR MOTION *(SHOULDER ADDUCTION)* | WRIST ROTATE (PRONATION) | - |
| PATTERN 3 | ANGULAR MOTION *(SHOULDER ABDUCTION)* | WRIST ROTATE (SUPINATION) | - |

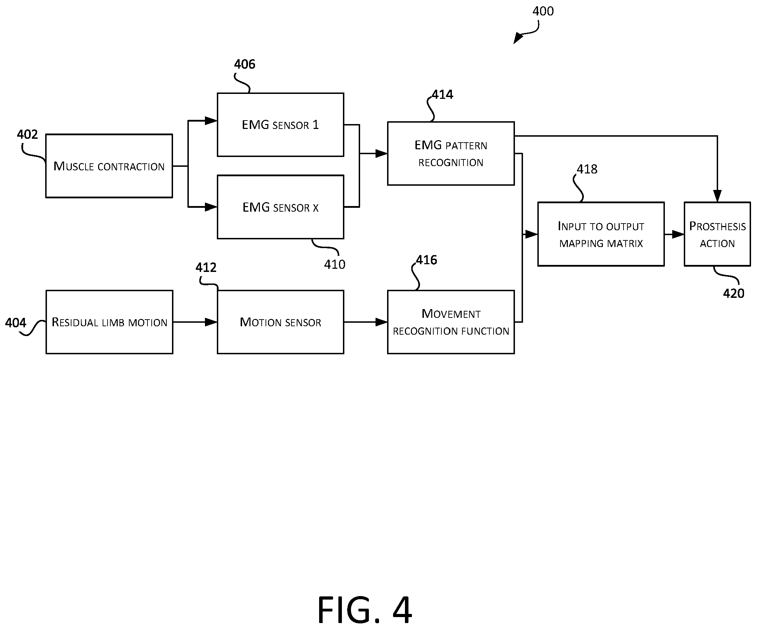

Fig. 4 is a block diagram of an embodiment of a control system 400 for controlling a prosthetic by mapping motion data and/or EMG data to a desired prosthesis action. The operation of the prosthesis may be based on multiple input signals. The wearer of the prosthesis may cause a muscle contraction 402 based at least in part on a desire to execute a prosthesis action 420. The one or more muscle contractions 402 may include a contraction of any one or more muscles. The prosthesis may map certain muscle contractions 402, which may or may not be in combination with other motion input data, to certain desired prosthesis actions 420.

In some embodiments, the muscle contraction 402 and the paired prosthesis action 420 may be predetermined. For instance, a bicep flexion be mapped to cause the opening of a prosthetic hand. In some embodiments, the muscle contractions 402 may be paired. For example, a biceps flexion may be mapped to one prosthesis action 420 and a biceps extension may be mapped to a different related prosthesis action 420. In other embodiments, one muscle contraction may be paired with a different muscle contraction. For example, a biceps flexion may be mapped to one prosthesis action 420 and a triceps flexion may be mapped to a different related prosthesis action 420.

The muscle contractions 402 and resulting EMG signals are detected by a plurality of EMG sensors. Two EMG sensors may be used. Any number of EMG sensors maybe used. As shown in Fig. 4, the operation of the prosthesis may include a plurality of EMG sensors including a first EMG sensor 406 and an Xth EMG sensor 410. The EMG sensors 406 and 410 may be configured to detect EMG signals generated by the muscle contractions 402.

The EMG sensors 406 and 410 may be configured to ignore muscle contractions that do not meet a preset threshold. For example, the system may require that the muscle contractions 402, and the corresponding electrophysiological signals, be at a certain level in order to be detected by the EMG sensors. In other implementations, the EMG sensors may detect lower level muscle contractions; however, the EMG sensors may not deliver the EMG signals to the EMG pattern recognition unless they reach a certain threshold. Some muscle contractions may not be acquired by the plurality of EMG sensors. The determination of whether the muscle contractions may be detected by the plurality of EMG sensors may depend on the location, sensitivity, calibration, and other features of the EMG sensors.

The EMG sensors 406 and 410 may produce one or more sets of EMG data corresponding to the one or more muscle contractions 402 which are then analyzed using an EMG pattern recognition 414, as seen in Fig. 6. The EMG pattern recognition 414 may be a memory module within a controller that causes a processor to analyze the EMG data. The EMG pattern recognition 414 may acquire and process the EMG data. The processing may include some signal processing, filtering, etc. The EMG pattern recognition 414 may determine patterns in the EMG signals. The EMG pattern recognition 414 may compare the one or more EMG signals to one or more thresholds to determine which of the one or more muscle contractions 402 satisfied the required thresholds for the prosthesis action 420. The EMG pattern recognition 414 may classify the EMG signals into one or more predetermined EMG patterns, wherein the patterns contain EMG signals of similar intensity, location, or any other factor. The analysis of the EMG data may be used alone to control the prosthesis action 420. The EMG data may be used in combination with analysis of motion data to control the prosthesis action 420.

In some embodiments, the EMG pattern recognition 414 may be extended to receive a residual limb motion 404 in addition to the EMG signals. The residual limb may experience unwanted EMG activity in certain situations. For example, where the wearer's prosthesis and limb are outstretched while holding an object, the lever force upon the residual limb may be large, due to the weight of the prosthesis and the object. The prosthesis may then receive unwanted EMG activity due to the muscles of the residual limb contracting to stabilize the prosthesis. The residual limb motion 404 may then be used to alert for unwanted EMG activity. The EMG pattern recognition 414 may acquire and process the EMG signals and the residual limb motion and determine patterns in the EMG signals and the residual limb motion.

The wearer of the prosthesis may cause a residual limb motion 404 based at least in part on a desire to execute the prosthesis action 420. The residual limb motion 404 may include angular and/or linear motion about and/or in the x, y, and/or z axes. In some embodiments, the residual limb motion 404 that is detectable by the motion sensor 412 may include a subset of these motions. In other embodiments, the residual limb motion 404 may include other motions. The residual limb motions 404 may be mapped to certain desired prosthesis actions 420. In some embodiments, the residual limb motion 404 and the paired prosthesis action 420 may be predetermined. For instance, a linear movement in the x-axis by the residual limb may cause a first prosthesis action and a linear movement in the y-axis by the residual limb may cause a second prosthesis action, etc. Any of the EMG and motion inputs described in Tables 1 and 2 may be used in the system 400.

The residual limb motion 404 is then detected by the motion sensor 412. One or more motion sensors 412 may detect motion data. The operation of the prosthesis may include a plurality of motion sensors, which may be configured to detect motion in different locations. In some embodiments, the operation of the prosthesis may include a plurality of motion sensors configured to detect different types of motion signals.

The motion sensor 412 may be any of the motions sensors described herein. For example, the motion sensor 412 may include at least one of or any combination of the following: accelerometers, gyroscopes, or magnetometers. In some embodiments, the motion sensor 412 may include other sensors configured to detect motion. The motion sensor 412 may include an IMS. The IMS may detect, measure, and/or report the motion relative to a starting location.

The motion sensor 412 may include a magnetic field sensor. The magnetic field sensor may detect, measure, and report the motion and/or orientation relative to a starting location and/or orientation of the sensor. The magnetic field sensor may be any of the magnetic field sensors described herein. The motion sensor 412 may include an IMU that includes one or more IMS's and one or more magnetic field sensors, such as a magnetometer. In some embodiments, the motions sensor 412 may only include one or more magnetic field sensors and no other type of motion sensor.

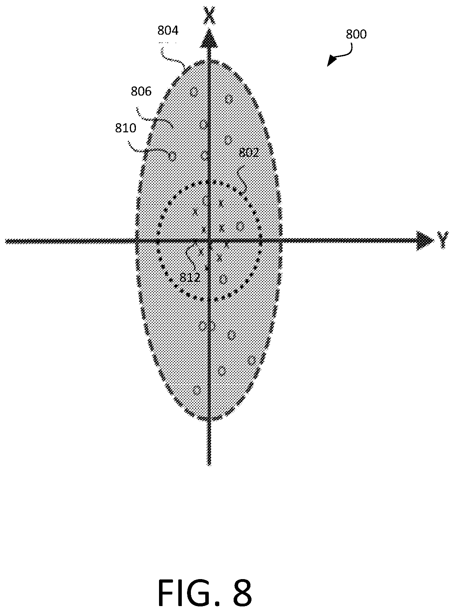

The motion sensor 412 may be configured to ignore motion that does not meet a preset threshold. As further described herein, for example with respect to Fig. 8, this threshold may be dynamic and change over time, for example as the wearer changes activities. In some embodiments, the threshold may be modified as the system learns the level of motion normally exhibited by the wearer. In some embodiments, the threshold may be manually changed by the wearer.

The motion sensor 412 may detect one or more motion signals corresponding to the one or more residual limb motions 404 for analysis by the movement recognition function 416 The movement recognition function 416 may be a memory module configured to cause a processor to analyze the motion data. The movement recognition function 416 may acquire and process the motion signals. The processing may include some signal processing, filtering, etc.

The movement recognition function 416 may detect trajectories and/or patterns in the one or more sets of motion data. The movement recognition function 416 may compare the motion data to one or more thresholds to determine if any and/or which of the one or more residual limb motions 404 satisfy the required thresholds for prosthesis action 420. The movement recognition function 416 may classify the motion data into one or more predetermined motion patterns, where the patterns contain motion signals of similar direction, magnitude, trajectory, intensity, location, and/or any other characteristic. In some embodiments, the movement recognition function 416 may be configured to deliver the one or more sets of motion data to a mapping matrix 418 only upon acknowledgement that the EMG pattern recognition 414 has received an EMG data pattern and/or classified an EMG data pattern. In some embodiments, the motion sensor 412 may be configured to deliver the one or more sets of motion data to the movement recognition function 416 only upon acknowledgement that the EMG pattern recognition 414 has received an EMG data pattern. The EMG signal pattern may be any of the patterns described herein, for example with respect to Figure 6. For example, the first EMG signal satisfies a first threshold, the second EMG signal satisfies a second threshold, both the first EMG signal and the second EMG signal satisfy respective thresholds, or any other EMG pattern.

The EMG pattern recognition 414 and the movement recognition function 416 may deliver the detected patterns to the input to output mapping matrix 418. The mapping matrix 418 may map the data, such as multiple patterns, detected by the EMG pattern recognition 414 and the movement recognition function 416. The activation of the first EMG sensor or the second EMG sensor may result in a first data or pattern or a second data or pattern, respectively. The activation of the first and the second EMG sensors simultaneously may result in a third data or pattern. The activation of the first and the second EMG sensors and the motion sensor simultaneously may result in a fourth data or pattern. The mapping matrix 418 may first detect actuation of the first and second EMG sensors before inquiring into the status of the motion sensor.

In other embodiments, the motion sensor may not require the activation of the first and second EMG sensors to generate data. For example, the activation of the motion sensor may result in a fifth data or pattern. In other embodiments, the activation of either the first EMG sensor or the second EMG sensor with the motion sensor may result in a sixth or seventh data or pattern, respectively. In other embodiments, the activation of the sensors may result in different patterns corresponding to different prosthesis actions. In other embodiments, one or more sensors may be added that may result in new patterns. In other embodiments, different combinations of different sensors being activated may result in different patterns. In other embodiments, the input to output mapping matrix 418 may implement mode-switching to detect more patterns.

The one or more outputs generated by the mapping matrix 418 may be analyzed by the prosthesis action 420. The prosthesis action 420 may be a memory module configured to cause a processor to generate a signal control a prosthesis, such as to actuate one or more prosthetic devices. The prosthesis action 420 may be configured to perform one or more prosthesis actions based on the output provided by the mapping matrix 418. The EMG pattern recognition 414 may deliver the results of analysis of the EMG data, such as intensity analysis, to the prosthesis action 420 which may then be used to determine the prosthesis action 420. In other embodiments, the movement recognition function 416 may deliver the results of analysis of the motion data, such as intensity analysis, to the prosthesis action 420 which may then be used to determine the speed of the prosthesis action 420. In some embodiments, both the EMG data and the motion data are analyzed and mapped for determining the prosthesis action 420.

Fig. 5 is a block diagram of an embodiment of a prosthetic system 500. Any of the features of the system 500 may be included in any of the other prostheses described herein. The system 500 includes one or more prosthetic devices and a control system for the one or more prosthetic devices where control is based on motion data and EMG data. The system 500 includes a residual limb 510 attached to a prosthesis 540. The prosthesis 540 may be a partial arm including an elbow, a partial arm not including an elbow, a hand, etc. The prosthesis 540 may include a plurality of prosthetic devices including any combination of a prosthetic hand 542A (which may include one or more powered digits), a prosthetic thumb 542B, a prosthetic wrist 542C, a prosthetic arm 542D, and a prosthetic elbow 542E. The prosthesis 540 may further include one or more sensor(s) 544, motor(s) 546, memory(ies) 548, controller(s) 552, and/or power supply(ies) 554. The residual limb 510 may be a portion of a sound arm. The prosthesis 540 may receive one or more EMG signals from one or more EMG device(s) 530 and one or more motion signals from one or more motion sensor device(s) 520.

Any combination of the prosthetic hand 542A, the prosthetic thumb 542B, the prosthetic wrist 542C, the prosthetic arm 542D, the prosthetic elbow 542E, the sensor(s) 544, the motor(s) 546, the memory(ies) 548, the controller(s) 552, the power supply(ies) 554, the motion sensor device(s) 520, and/or the EMG device(s) 530 may be in electrical communication with each other. For instance, the controller(s) 552 may communicate with any of the motor(s) 546, the sensor(s) 544, power supply(ies) 554 and/or motion sensor device(s) and EMG device(s). Depending on the embodiment, the prosthetic hand 542, the prosthetic thumb 542B, the prosthetic wrist 542C, the prosthetic arm 542D, the prosthetic elbow 542E, the sensor(s) 544, the motor(s) 546, the memory(ies) 548, the controller(s) 552, and/or the power supply(ies) 554 may be located on the prosthesis 540, and/or in including any location on the residual limb 510, remote from the residual limb 510, or attached to the wearer of the prosthesis 540. Some of these separate components may be combined in a variety of ways to achieve particular design objectives. For example, in some embodiments, the prosthetic elbow 542E may be combined with the prosthetic arm 542D to save cost and/or improve performance. Furthermore, the prosthesis 540 may include fewer or more components, as desired.

The EMG device(s) 530 may be located in a number of locations including any location at which muscle contractions or other bodily EMG signals may be detected, such as the skin, surface, tissue, other suitable locations, or combinations thereof. The EMG device(s) may be located throughout the body of the wearer. The motion sensor device(s) may be located in a number of locations including any location at which motion may be detected. For example, the motion sensor device(s) 520 may be located on the residual limb 510 and may provide information related to the motion of the residual limb 510. At least one of the EMG device(s) 530 and the motion sensor device(s) 520 may provide signals indicative of received EMG signal(s) and motion signal(s) to the prosthesis 540.

The prosthesis 540 may include one or more motor(s) 546 for moving the prosthetic hand 542A, the prosthetic thumb 542B, the prosthetic wrist 542C, the prosthetic arm 542D, and/or the prosthetic elbow 542E. For example, in some embodiments, the prosthesis 540 includes a separate motor for each prosthetic device include in the prosthesis 540 including a separate motor for the prosthetic hand 542A and a separate motor for the prosthetic wrist 542C etc. The prosthesis 540 may include any number of motor(s) 546 and may provide actuation from the motor(s) 546 to the prosthetic devices proportionally or non-proportionally. For example, the prosthesis 540 may provide four motors for the prosthetic arm 542D and one motor for the prosthetic thumb 542B. The one or more motor(s) 546 of the prosthesis 540 may be individually or collectively referred to as motor 546, motor(s) 546, or motors 546.

The prosthesis 540 may include or be in communication with one or more sensor(s) 544, which may individually or collectively be referred to as sensor 544, sensor(s) 544, or sensors 544. The sensor(s) 544 may be located in any one or more locations, including any location in or on the residual limb 510, the prosthesis 540, or remote from the residual limb 510 or prosthesis 540. The sensors 544 may capture information relating to position, speed, acceleration, orientation, torque, current, voltage, force, or movement of the prosthesis 540. The sensor data may be processed in real-time by the sensor(s) 544 or a processing device of the prosthesis 540, such as the controller 552. The sensor(s) 544 may include, but are not limited to, one or more torque sensors, current sensors, voltage sensors, force sensors, acceleration or orientation sensors, or position sensors. The sensor(s) 544 may be located in any number of locations in or on the prosthesis 540, a wearer of the prosthesis 540, or remote from the prosthesis 540.

A torque sensor of the sensor(s) 544 may capture information relating to a torque of the motor(s) 546. A current sensor of the sensor(s) 544 may capture information relating to one or more currents flowing through the prosthesis 540, such as a current drawn by the motor(s) 546 a current flowing from the power supply 554. A voltage sensor of the sensor(s) 546 may capture information relating to one or more voltages of the prosthesis 540, such as a voltage received by a motor(s) 546 or a voltage of the power supply 554.

For example, a torque sensor may be configured to measure a component of force applied to the prosthesis 540 from the ground or other supporting surface in a direction substantially along or parallel to a shin longitudinal axis. In some cases, the force sensor may be implemented as a load cell.

Data from the torque, voltage, or current sensors may be received by the controller(s) 552 may used to determine various parameters associated with the prosthesis 540, such as a torque of the motor(s) 546 or whether a motor-stall-threshold is satisfied.

An acceleration or orientation sensor of the sensor(s) 544 may capture information relating to position, speed, acceleration, or orientation of the prosthesis 540, such as position, speed, acceleration, or orientation data relating to any of the prosthetic hand 542A, the prosthetic thumb 542B, the prosthetic wrist 542C, the prosthetic arm 542D, and the prosthetic elbow 542E. In some instances, the acceleration or orientation sensor may capture information corresponding to in multiple axes, such as two or three substantially mutually perpendicular axes. In some embodiments, the acceleration or orientation sensor may be implemented as one or more of an accelerometer, an orientation sensor, a gravity sensor, or a gyroscope.

Data from the acceleration or orientation sensor may be received by the controller(s) 552 may used to determine various parameters associated with the prosthesis 540, such as an acceleration of the prosthetic hand 542A, a deceleration of the prosthetic hand 542A, an orientation of the prosthetic hand 542A, an orientation of the prosthetic elbow 542E, a torque of a motor 546, a position of the prosthetic thumb 542B, or the like.

A force sensor of the sensor(s) 544 may capture information relating to a force applied on or by any of the prosthetic hand 542A, the prosthetic thumb 542B, the prosthetic wrist 542C, the prosthetic arm 542D, and the prosthetic elbow 542E. For example, in some cases, the prosthetic thumb 542B or portions thereof may be fitted with capacitive or inductive force sensors. The force sensor may be configured to measure a component of force applied to the prosthetic thumb 542B by an object or other external force in one or more directions. In some cases, the force sensor may be implemented as a load cell.

Force measurement data from the force sensor may be received by the controller(s) 552 and may be used to determine various parameters associated with the prosthesis 540, such as a force applied to the prosthetic thumb 542B, a torque of a motor 546, a position of the prosthetic arm 542D, or the like. For example, using the force measurement data, the controller 552 may determine whether the prosthetic thumb 542B is touching an object or other opposing force as it is moved by a motor 546.

A position sensor of the sensor(s) 544 may capture information relating to position of the prosthetic devices located in the prosthesis, such as an absolute position of the prosthetic thumb 542B. In some embodiments, the position sensor of the sensor(s) 544 may be implemented as a Hall Effect sensor. For example, the motor 546 or the prosthetic thumb 542B may include a magnet, such as a magnet about 0.5 to 5 mm in diameter or about 1 mm or 2 mm in diameter, and the magnet may be positioned on a rotating link of the motor 546 or the prosthetic thumb 542B. As the link rotates, the distance between the magnet and the Hall Effect sensor changes. The Hall Effect sensor may sense the magnet, and the Hall Effect sensor may provide signals with different levels of current output depending on the proximity of a magnetic field, which changes as the distance from the magnet changes. By calibrating the variation of the signalled current by the Hall Effect sensor versus an associated angle that the prosthetic thumb 542B or portions thereof rotates, the controller 552 may use the signalled current by the Hall Effect sensor to determine the angular position of the prosthetic thumb 542B or portions thereof.

In some embodiments, the position sensor of the sensor(s) 544 may be implemented as a potentiometer, which may be used to obtain the absolute position of at least one of the prostheses in the prosthesis 540. In some embodiments, an incremental optical rotary encoder and/or gyro sensor may be used to control at least one of the prostheses in the prosthesis 540. For example, an incremental optical rotary encoder may generate a signal when a motor 546 moves. The motor 546 may include absolute optical encoders. For example, the motor 546 may include absolute optical encoders that monitor an internal position of the motor 546. In some embodiments, the controller 552 may derive the position of the prosthetic arm 542D based at least in part on the motor's rotation.

Position data from the position sensor may be received by the controller(s) 552 may used to determine various parameters associated with the prosthesis 540, such as an absolute position, a relative position, or an angular position of the prosthetic wrist 542C.

The prosthesis 540 may include or be in communication with one or more EMG sensor device(s) 530 and one or more motion sensor device(s) 520. The motion sensor device(s) 520 and the EMG device(s) 530 may capture or receive user input and may transmit signals to the prosthesis 540, such as the controller 552, based at least in part on the user input. The motion sensor device(s) 520 may capture or receive user input relating to a motion and a corresponding intensity of motion. The EMG device(s) 530 may capture or receive user input relating to an electrophysiological signal, i.e. a muscle contraction, and a corresponding intensity of contraction.

The motion sensor device(s) 520 and the EMG device(s) 530 may relay information associated with the respective activity to the controller(s) 552. The relayed information may be in the form of one or more activation signals. The controller(s) 552 may communicate command signals to the motor(s) 546 based at least in part on the one or more activation signals received from the motion sensor device(s) 520 and the EMG device(s) 530. The motor(s) 546 may actuate at least one of the prosthetic hand 542A, the prosthetic thumb 542B, the prosthetic wrist 542C, the prosthetic arm 542D, and/or the prosthetic elbow 542E based on receiving the command signals from the controller(s) 552.

The power supply(ies) 554 may be electrically coupled and/or supply power to the motor(s) 546. As described herein, the motor(s) 546 uses energy supplied by the power supply 554 to move the prosthetic hand 542A, the prosthetic thumb 542B, the prosthetic wrist 542C, the prosthetic arm 542D, and/or the prosthetic elbow 542E. In some embodiments, the power supply(ies) 554 may include a battery. For example, a battery of the power supply(ies) 554 may include one or more battery cells arranged in series or parallel, such as, but not limited to, 2, 4, 6, or 8 cells. Furthermore, in some cases, a battery of the power supply(ies) 554 may have a high-energy density. For example, a battery of the power supply(ies) 554 may include Lithium-ion (Li-ion), Lithium Polymer (Li-Pol), or the like. Specifications of the power supply 554 may vary across embodiments. For example, a power supply may be selected which fulfills power supply requirements of the motor(s) 546. In some embodiments, the power supply(ies) 554 may have a nominal voltage between 5 V and 20 V, such as a nominal voltage of 7.4 V. However, the power supply(ies) 554 may be appropriately sized or rated based on power supply requirements of the prosthesis 540.

The motor(s) 546 may be a brushed DC motor or a brushless motor. In some embodiments, a motor 546 may be implemented as an electric rotary actuator. However, other types of motors or actuators may be used without departing from the scope of the claims. In some embodiments, the motor(s) 546 may be controlled using pulse width modulation. For example, the controller(s) 552 may supply or cause another component of the prosthesis 540 to supply a pulse width modulated signal to the motor(s) 546 to control movement of at least one of the prosthetic hand 542A, the prosthetic thumb 542B, the prosthetic wrist 542C, the prosthetic arm 542D, and/or the prosthetic elbow 542E.

Fig. 6 is a plot 600 illustrating an embodiment of an EMG data profile that may be used in the various control systems and methods described herein The plot 600 illustrates an example minimum muscle contraction classification algorithm for the EMG pattern recognition 414, according to some embodiments. The vertical axis on the plot 600 corresponds to a measure of EMG activity in the first channel or sensor, measured in microvolts. The horizontal axis on the plot 600 corresponds to a measure of EMG activity in second channel or sensor, measured in microvolts. In the illustrated example, the horizontal axis and the vertical axis have a range of 1200 microvolts and 1400 microvolts respectively.

The plot 600 illustrates example EMG inputs by two EMG sensors to the prosthesis, for example a controller thereof, over a time period. The time period may be from about 50-500 milliseconds, from about 100-300 milliseconds, or from about 150-200 milliseconds, or for shorter or longer time periods. The plot 600 illustrates various signal groupings that have been grouped according to the various patterns that they represent. The groupings may correlate to muscle contraction patterns. The first grouping 602 represents the first pattern. The first grouping 602 correlates to the inputs that have a sufficient y-axis input, first EMG channel, but have an insufficient x-axis input, second EMG channel. The second grouping 604 represents the second pattern. The second grouping 604 correlates to the inputs that have a sufficient x-axis input, second EMG channel, but have an insufficient y-axis input, first EMG channel.

Both the first grouping 602 and the second grouping 604 may have thresholds that must be satisfied for an input to fall within the first grouping 602 or the second grouping 604. When the inputs fail to meet either of these thresholds, the EMG sensors are considered to be at rest and no prosthesis action may be performed. As seen in the example for the first EMG channel, the threshold voltage is roughly 200 microvolts and 450 microvolts for the second EMG channel. In some embodiments, the minimum thresholds for the first EMG channel and the second EMG channel may be the same. In other embodiments, the minimum thresholds for the first EMG channel and the second EMG channel may be different. The inputs that fall outside of these thresholds may be seen in the example as the inputs not covered by the first grouping 602, the second grouping 604, or the third grouping 606. The inputs not covered by the first grouping 602, the second grouping 604, or the third grouping 606 may correlate to the prosthesis being at rest. The first grouping 602 may correlate to a first prosthesis action. In some embodiments, the first grouping 602 may correlate to the actuation of one or more prostheses. In other embodiments, the first grouping 602 may correlate to a coordinated preposition where the motor may actuate the prosthesis so that the prosthesis is moved to a predetermined position. This movement may be based upon detection of the first grouping 602. The second grouping 604 may correlate to a second prosthesis action. In some embodiments, the second grouping 604 may correlate to the actuation of one or more prostheses. In other embodiments, the second grouping 604 may correlate to a coordinated preposition where the motor may actuate the prosthesis so that the prosthesis is moved to a predetermined position. This movement may be based upon detection of the second pattern.

In the example shown in Fig. 6, the third grouping 606 correlates to the first EMG channel reaching a certain threshold and the second EMG channel reaching another threshold. As seen in the example, to qualify for the third grouping 606, the first EMG channel has a threshold voltage of roughly 500 microvolts and the second EMG channel has a threshold voltage of roughly 400 microvolts. When both of these thresholds have been satisfied, the third grouping 606 has been satisfied. In some embodiments, the minimum thresholds for the first EMG channel and the second EMG channel may be the same. In other embodiments, the minimum thresholds for the first EMG channel and the second EMG channel may be different. The third grouping 606 may correlate to a third prosthesis action. In some embodiments, the third grouping 606 may correlate to the actuation of one or more prosthetic devices. In other embodiments, the third grouping 606 may correlate to a coordinate preposition where the motor may actuate the prosthesis so that the prosthesis is moved to a predetermined position. The movement may be based upon detection of the third pattern.

Fig. 7 is a data plot 700 illustrating an embodiment of an EMG and motion data profile that may be used in the various control systems and methods described herein. The plot 700 illustrates an example motion sensor and muscle contraction classification algorithm for the input to output mapping matrix 418, according to some embodiments. The vertical axis on the plot 700 corresponds to a measure of the intensity of the motion sensor data received by the motion sensor. The motion sensor, in this example, is a gyroscope and is measuring angular velocity which here is measured in units of degrees per second. In other embodiments, the motion sensor may be an accelerometer, a magnetic field sensor, or any other sensor configured to detect motion. The units of measurement may be the corresponding units configured to measure motion, such as meters per second, magnetic field orientation, etc. The horizontal axis on the plot 700 corresponds to a measure of EMG activity in the first and second EMG channels or sensors measured in microvolts. In the illustrated example, the horizontal axis and the vertical axis have a range of 0-1200 microvolts and 0-0.14 degrees per second (angular displacement) respectively. Other ranges and/or data may be used. The motion sensor vertical axis data may be angular displacement, linear displacement, angular acceleration, linear acceleration, total distance travelled, trajectory travelled, other suitable motion sensor data, or combinations thereof.

The plot 700 illustrates one example motion sensor input and two example EMG sensor inputs to the prosthesis over a time period. In this example, the time period may be a minute, 10 minutes, an hour etc. The plot 700 illustrates various groupings that have been grouped according to the various patterns that they represent. The first grouping 702 represents the first pattern. The first grouping 702 correlates to the inputs that have a sufficient y-axis input, motion sensor channel, but have an insufficient x-axis input, first and second EMG channel. The first grouping 702 may correlate to a translation, rotation, motion pattern, etc. The second grouping 704 represents the second pattern. The second grouping 704 correlates to the inputs that have a sufficient x-axis input, first and second EMG channels, but have an insufficient y-axis input, motion sensor channel.

Both the first grouping 702 and the second grouping 704 may have thresholds that must be satisfied for an input to fall within the first grouping 702 or the second grouping 704. In some embodiments, the second grouping 704 correlates to the third grouping 606 in Figure 6. When the inputs fail to meet either of these thresholds, the sensors are considered to be at rest and no prosthesis action may be performed. In some embodiments, the first grouping 604 and the second grouping 606 may still perform a correlating prosthesis action. As seen in the example for the motion sensor channel, the threshold velocity is roughly 0.02 degrees per second and 450 microvolts for the second EMG channel. The inputs that fall outside of these thresholds may be seen in the example as the inputs not covered by the first grouping 702, the second grouping 704, or the third grouping 706. The first grouping 702 may correlate to a first prosthesis action. In some embodiments, the first grouping 702 may correlate to the actuation of one or more prosthesis. In other embodiments, the first grouping 702 may correlate to a coordinated preposition where the motor moves the prosthesis. The movement may be based upon detection of the first grouping 702. The second grouping 704 may correlate to a second prosthesis action. In some embodiments, the second grouping 704 may correlate to the actuation of one or more prosthesis. In other embodiments, the second grouping 704 may correlate to a coordinated preposition where the motor moves the prosthesis. The movement may be based upon detection of the second pattern.

In the example shown in Fig. 7, the third grouping 706 correlates to the first EMG channel, the second EMG channel, and the motion sensor channel each reaching a certain threshold. In some embodiments, each threshold may be similar or the same. In other embodiments, each threshold may be different. In other embodiments, the third grouping 706 may correlate to one of the first EMG channel and the second EMG channel reaching a certain threshold with the motion sensor channel reaching a different threshold. As seen in the example, to qualify for the third grouping 706, the motion sensor channel has a threshold velocity of roughly 0.05 degrees per second and the first and second EMG channels have a threshold voltage of roughly 400 microvolts. When both of these thresholds have been satisfied, the third grouping 706 has been satisfied. The third grouping 706 may correlate to a third prosthesis action. In some embodiments, the third grouping 706 may correlate to the actuation of one or more prosthetic devices. In other embodiments, the third grouping 706 may correlate to a coordinate preposition where the motor moves the prosthesis. The movement may be based upon detection of the third pattern.

Fig. 8 is a data plot 800 illustrating an embodiment of a dynamic movement threshold for a motion sensor that may be used in the various control systems and methods described herein. The plot 800 illustrates an example movement threshold for the motion sensor. The motion sensor may detect any motion and deliver a corresponding signal to the prosthesis. However, the prosthesis interprets the motion and decides whether the motion corresponds to a deliberate motion with a corresponding prosthesis action or whether the motion is analogous to background noise. Dynamic movement thresholds may be implemented to ensure that the wearer may activate the deliberate motions to implement the corresponding prosthesis actions while in motion e.g. while the wearer is walking, running, climbing stairs, etc. The prosthesis may increase the movement threshold dynamically such that movements that would have otherwise met the original movement threshold, such as the movement associated with the wearer walking, no longer meet the increased threshold. The prosthesis may detect the occurrence of a movement event based upon a repeated input by the motion sensor. In some embodiments, the prosthesis may detect the occurrence or the entry of a movement event based upon wearer input.

The angular and linear movement of the residual limb may be monitored by the motion sensor of the prosthesis. The angular and linear movement of the residual limb may be monitored in all axes. In some implementations, the angular and linear movement of the residual limb may be monitored in fewer than all of the axes. For example, the angular movement may only be monitored in the x-axis and the linear movement may only be monitored in the x-axis and z-axis. In the example shown in the plot 800, the residual limb is being monitored for angular movement in the x-axis and the y-axis. The original threshold 802 represents the default or original movement threshold. The original threshold 802 represents the threshold level of motion required for the location of the motion sensor to be considered in motion when the wearer has not entered into a movement event.

The prosthesis, e.g. a controller thereof, may elect to change from the original threshold 802 to the dynamic threshold 804 based on the initialization of a movement event. Movement events may be monitored by the prosthesis and the detection of a movement event may result in an increase to the movement threshold in the affected axes. In some examples, the movement event may only effect the movement threshold in one axis. In some embodiments, the dynamic threshold 804 may be a lower threshold and motion sensor inputs that did not meet the original threshold 802, may meet the dynamic threshold 804. For example, if the wearer is unable to generate motion that meets the original threshold 802, the prosthesis may implement a dynamic threshold 804 that requires less movement. The prosthesis may be configured to detect when an activity has been entered based upon movement patterns associated with that activity. In some embodiments, the user may provide some input to the prosthesis via a button, GUI, or some other device that informs the prosthesis that an activity has been entered and the movement threshold should be changed. The dynamic threshold 804 represents an updated threshold where the dynamic threshold 804 is a modified or updated version of the original threshold 802. The dynamic threshold 804 represents the threshold level of motion required for the wearer to be considered in motion while doing an activity or movement event. In some embodiments, the dynamic threshold 804 might correlate to one or more activities. In some embodiments, different activities might have different dynamic thresholds. For example, running might have a different dynamic movement threshold than walking as running may be associated with more movement of the residual limb.

The area within the original threshold 802 represents the inputs of the motion sensors that will result in the prosthesis being inactive when in the original state. In this example, the area within the original threshold 802 is also within the dynamic threshold 804 and represents inputs of the motion sensors that will result in the prosthesis being inactive when in the original state or the dynamic state. The first inputs 812 represent motion sensor inputs during the original state that do not meet the original threshold 802. The inputs 812 represent when the wearer's motion sensor is at rest and the motion data is clustered within the original threshold 802. In other embodiments, the motion sensor may be placed on the residual limb of the wearer, the prosthesis, or at any location sufficient to capture motion information. The inactive area 806 within the dynamic threshold 804 represents the inputs of the motion sensors that will result in the prosthesis being inactive when in the dynamic state. In this example, the inactive area 806 also includes the area within the original threshold 802. The second inputs 810 represent motion sensor inputs during the dynamic state that do not meet the dynamic threshold 804. The inputs 810 represent when the wearer begins to walk and the angular movement data becomes stretched along the x-axis due to the natural pendulum swing of the user's arm. The original threshold 802 is modified to the dynamic threshold 804 to account for the natural movement of the wearer while in motion. In this example, the user's arm is moving more in the x-axis than in the y-axis while walking and the dynamic threshold 804 denotes this. In other examples, the x-axis and the y-axis may be affected equally. The wearer may be able to modify the original threshold 802 and the dynamic threshold 804 to account for increased or decreased levels of movement. The wearer may also be able to modify the movement gestures required to actuate a prosthesis action when moving.

Fig. 9 is a perspective view of an embodiment of a prosthetic hand control profile 900 illustrating various movements that may be detected by a motion sensor that may be used in the various control systems and methods described herein. The profile 900 illustrates an example of a proposed control algorithm for an example motion sensor. In the profile 900, the example motion sensor may be an inertial measurement sensor (IMS) and/or magnetic field sensor. In some embodiments, the motion sensor may be any other motion sensor including a magnetic field sensor. Using an IMS as an example motion sensor for illustrative purposes, the IMS may be placed on the prosthetic. In other embodiments, the IMS may be placed on the residual limb or at any other location located on the wearer or off the wearer. The IMS may be part of an IMU that consists of any one or more of the following: an accelerometer, a gyroscope, and a magnetometer. In the profile 900, the IMS consists of an accelerometer and a gyroscope. The accelerometer may track linear acceleration. In the present example, the accelerometer is tracking the linear acceleration of the residual limb. The gyroscope may track angular velocity. In the present example, the gyroscope is tracking the angular velocity of the residual limb. A magnetometer may be used to track absolute orientation relative to the earth's magnetic field.

In the profile 900, the proposed control algorithm detects and classifies movement in the angular and linear planes of motion. In this example, the movement may be in the prosthetic or residual limb, however, in other examples, the proposed control algorithm may be configured to detect motion at any other location. In some embodiments, the proposed control algorithm may detect motion in one plane of motion. In other embodiments, the proposed control algorithm may be configured to detect motion in planes of motion other than the linear and angular planes of motion. In the present example, the proposed control algorithm may require the wearer perform a linear or angular movement or gesture of the residual limb along the x-axis, the y-axis, or the z-axis. Based upon the choice of plane of motion and the choice of axis, the proposed control algorithm may be configured to implement a correlating prosthesis action. In the present example, the wearer is provided with 12 motion-based input gestures that may correspond to 12 different prosthesis actions. In other embodiments, the prosthesis may employ more complex pattern recognition algorithms to detect and classify more advanced residual limb motions. For example, the pattern recognition algorithm may be applied to recognize more complex movements consisting of multiple combinations of angular velocity and linear acceleration.

Various "gesture control" and/or other prosthetic structural or control features may be implemented in any of the embodiments described herein, for example those features described in U.S. Patent No. 6,361570, filed April 24, 2000, and titled "UPPER LIMB PROSTHESIS," in U.S. Patent No. 8,808,397, filed Oct. 16, 2009, and titled "PROSTHESES WITH MECHANICALLY OPERABLE DIGIT MEMBERS," in U.S. Patent No. 8,657,887, filed Oct. 05, 2010, and titled "PROSTHESIS COVERING," in U.S. Patent No. 8,828,096, filed Aug. 09, 2011, and titled "PROSTHESIS COVERING," in U.S. Patent No. 9,402,749, filed May 10, 2012, and titled "METHOD OF CONTROLLING A PROSTHESIS," in U.S. Patent No. 8,986,395, filed Oct. 31, 2012, and titled "HAND PROSTHESIS," in U.S. Patent No. 9,278,012, filed April 29, 2014, and titled "PROSTHESIS OR AN ORTHOSIS AND A METHOD FOR CONTROLLING A PROSTHESIS OR AN ORTHOSIS," in U.S. Patent No. 10,398,576, filed May 13, 2014, and titled "PROSTHETIC FEEDBACK APPARATUS AND METHOD," in U.S. Patent No. 9,463,100, filed April 29, 2014, and titled "METHOD AND APPARATUS FOR CONTROLLING A PROSTHETIC DEVICE," in U.S. Patent No. 8,696,763, filed Jan. 26, 2012, and titled "PROSTHETIC APPARATUS AND CONTROL METHOD," in U.S. Patent No. 8,995,760, filed May 21, 2012, and titled "METHOD AND APPARATUS FOR COLOURING A COSMETIC COVERING," in U.S. Patent Application No. 14/765638, filed Aug. 04, 2015, and titled "MULTI-MODAL UPPER LIMB PROSTHETIC DEVICE CONTROL USING MYOELECTRIC SIGNALS," in U.S. Patent No. 9,387,095, filed Jan. 23, 2015, and titled "PROSTHETICS AND ORTHOTICS," in U.S. Patent No. 8,197,554, filed Feb. 13, 2009, and titled "ROTARY ACTUATOR ARRANGEMENT," in U.S. Patent No. 9,999,522, filed Aug. 23, 2016, and titled "PROSTHETIC DIGIT FOR USE WITH TOUCHSCREEN DEVICES," in U.S. Patent No. 10,265,197, filed Oct. 28, 2016, and titled "SYSTEMS AND METHODS FOR CONTROLLING A PROSTHETIC HAND," in U.S. Patent No. 10,449,063, filed March 01, 2017, and titled "WRIST DEVICE FOR A PROSTHETIC LIMB," in U.S. Patent No. 10,369,024, filed Nov. 02, 2016, and titled "SYSTEMS AND METHODS FOR PROSTHETIC WRIST ROTATION," or in U.S. Patent No. 9,839,534, filed Feb. 04, 2015, and titled "MODULAR AND LIGHTWEIGHT MYOELECTRIC PROSTHESIS COMPONENTS AND RELATED METHODS,". It is however noted, that none of these patents and patent applications form part of the scope of protection of the present invention.

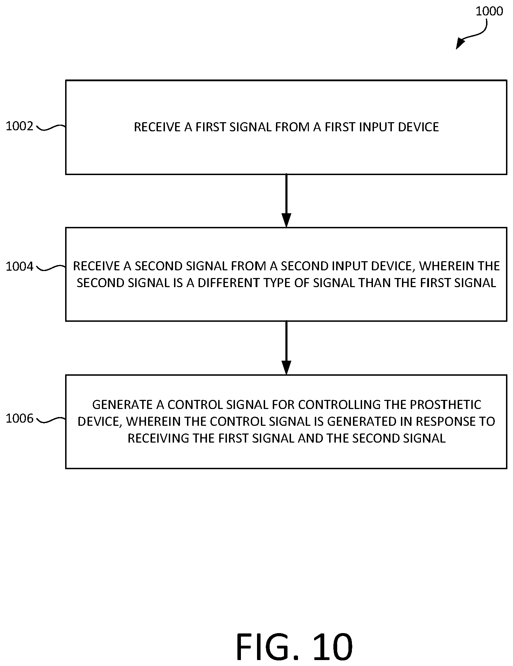

Fig. 10 is a flow diagram showing an embodiment of a method 1000 for controlling a prosthetic device based on EMG and motion data. The method 1000 may be used for a prosthesis control algorithm receiving both EMG and motion sensor inputs that is configured to actuate a prosthesis based at least in part on the received EMG and motion sensor inputs. The elements outlined for method 1000 may be implemented by one or more computing devices associated with the prosthesis, such as the controller(s) 552, and one or more motor(s) 546 associated with the prosthesis. Accordingly, method 1000 has been logically associated as being generally performed by the controller(s) 552 and the motor(s) 546. However, the following illustrative embodiment should not be construed as limiting. Any of the prostheses described herein may be used to perform the method 1000.

At block 1002, the controller(s) 552 receives a first signal from a first input device. The first input device may be a myoelectric electrode or EMG sensor. In some implementations, the first input device may be any other device configured to detect muscle contractions. The myoelectric electrode may detect electric activity from a muscle of a wearer of the prosthesis. In some configurations, the myoelectric electrode may detect electric activity from a muscle contraction of a residual limb of the wearer of the prosthesis. The myoelectric electrode may deliver an EMG signal to the controller(s) 552. In some embodiments, the controller(s) 552 may receive a plurality of EMG signals from a plurality of myoelectric electrodes. The plurality of EMG signals from a plurality of myoelectric electrodes may detect electric activity from a plurality of muscles of the wearer of the prosthesis.

At block 1004, the controller(s) 552 receive a second signal from a second input device, wherein the second signal is a different type of signal than the first signal. The second input device may be a motion sensor configured to detect motion at a certain location. In some embodiments, the motion sensor may be an inertial measurement sensor configured to measure inertial measurement data associated with a motion. The inertial measurement sensor may contain one or more of the following: an accelerometer, a gyroscope, and a magnetometer. In some embodiments, the motion sensor may be a magnetic field sensor configured to measure magnetic field data associated with a motion. In some implementations, the controller(s) receive a plurality of second signals from a plurality of second input devices. In some implementations, the controller(s) receive at least IMU data from an IMS and magnetic field data from a magnetic field sensor. The motion sensor may be located on the residual limb of the wearer. In other implementations, the motion sensor may be located on the prosthesis of the wearer. In other implementations, the motion sensor may be located at other locations on or off the wearer.

At block 1006, the controller(s) 552 generate a control signal for controlling the prosthesis. The control signal may be based at least in part on the first signal and the second signal. As seen in Figures 6 and 7, the controller(s) 552 may reference a control algorithm for determining the prosthesis action related to the inputs of the first and second signals.

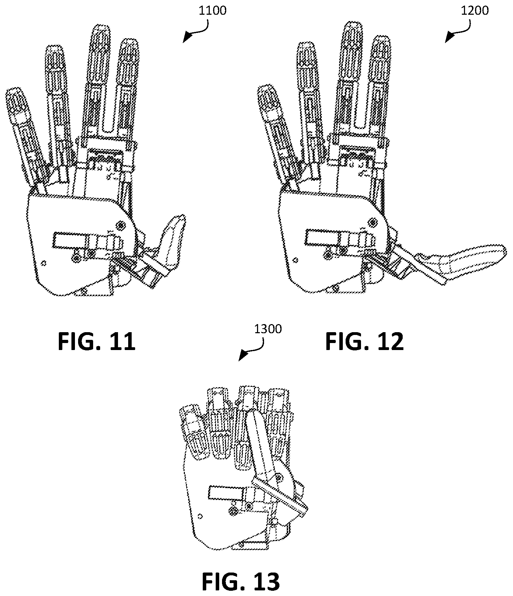

Figs. 11-13 are various front views of a hand prosthetic shown in various configurations after being controlled using the various control systems and methods described herein. Each of Figs. 11-13 represent possible prosthesis actions based upon inputs to the prosthesis. After receiving the input signals, for example two EMG signals and one motion sensor signal in the present embodiment, the controller(s) 552 enter a device configuration stage. During the device configuration stage, each of the valid input combinations of EMG signals and motion sensor signal are logically mapped to a specified prosthesis output. In some embodiments, the valid input combinations may include receiving EMG signals without receiving the motion sensor signal. In other embodiments, the valid input combinations may include receiving the motion sensor signal without receiving the EMG signals.

The wearer of the prosthesis may be provided with information indicating a relationship between a valid input combination and a specified prosthesis action. In some implementations, the wearer of the prosthesis may be provided with an output of each relationship between each valid input combination and each corresponding specified prosthesis action. In some implementations, this may be modifiable by the wearer. For example, the wearer may elect to map a combination of motion sensor signal and EMG signals to a different prosthesis action.

The prosthesis action may be defined in two forms. The first form of the prosthesis action may be direct control. Direct control may involve actuating one degree of freedom of one prosthetic device of the prosthesis. For example, direct control may include flexion/extension of the elbow, supination/pronation of the wrist rotator, palmar/lateral rotation of the thumb, etc. Direct control may allow the wearer to have direct control of the independent degrees of freedom of the prosthetic devices of the prosthesis without the need for mode switching. As seen in Figs. 11 and 12, direct control may involve moving the powered thumb from the relaxed position 1100, as shown in Fig. 11, to the palm-flat position 1200, as shown in Fig. 12. The second form of the prosthesis action may be coordinated prepositioning. Coordinated prepositioning may involve actuating one or more prosthetic devices of the prosthesis simultaneously and automatically prepositioning them to a pre-defined posture. In some implementations, coordinated prepositioning may involve formation of a grip with the prosthetic hand, rotation of a prosthetic digit, rotation of the prosthetic wrist, and/or rotation of the prosthetic elbow. As seen in Figs. 11 and 13, coordinated prepositioning may involve moving from the relaxed position 1100, as shown in Fig. 11, to the cylindrical grip 1300, as shown in Fig. 13, where the cylindrical grip 1300 is a pre-defined posture. In other implementations, the prosthesis action may be defined in more or less forms.

Various modifications to the implementations described in this disclosure can be readily apparent to those skilled in the art, and the generic principles defined herein can be applied to other implementations without departing from the scope of the claims. Thus, the disclosure is not intended to be limited to the implementations shown herein, but is to be accorded the widest scope consistent with the claims, the principles and the novel features disclosed herein. The word "example" is used exclusively herein to mean "serving as an example, instance, or illustration." Any implementation described herein as "example" is not necessarily to be construed as preferred or advantageous over other implementations.

Certain features that are described in this specification in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing can be advantageous. Moreover, the separation of various system components in the implementations described above should not be understood as requiring such separation in all implementations, and it should be understood that the described program components and systems can generally be integrated together in a single software product or packaged into multiple software products. Additionally, other implementations are within the scope of the following claims. In some cases, the actions recited in the claims can be performed in a different order and still achieve desirable results.

It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. **In** addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). **In** those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B".

## Claims

1. A method of controlling an upper limb prosthetic device, the method comprising:
receiving electromyography (EMG) data generated by an EMG sensor (104) in response to a muscle contraction of a residual limb (510) of a user of the prosthetic device,
receiving, in response to a motion of the residual limb (510) or of the prosthetic device, at least one of i) inertial measurement data generated by one or more inertial measurement sensors (IMS) or ii) magnetic field data generated by one or more magnetic field sensors (210);
analyzing the EMG data and the at least one of the inertial measurement data or the magnetic field data using a mapping matrix (418); and
generating a control signal for controlling the prosthetic device, wherein the control signal is generated in response to analyzing the EMG data and the at least one of the inertial measurement data or the magnetic field data.

2. The method of Claim 1, wherein the motion comprises a motion pattern, a translation, or a rotation.

3. The method of Claim 1, wherein the muscle contraction comprises a muscle contraction pattern.

4. The method of Claim 1, further comprising entering a control mode for the prosthetic device in response to receiving the EMG data, and then receiving the at least one of the inertial measurement data or the magnetic field data.

5. The method of Claim 1, further comprising monitoring movement of the residual limb (510) to generate a movement threshold, wherein generating the control signal comprises comparison of the at least one of the inertial measurement data or the magnetic field data with the movement threshold.

6. The method of Claim 5, further comprising replacing the movement threshold with an updated movement threshold, wherein generating the control signal comprises comparison of the at least one of the inertial measurement data or the magnetic field data with the updated movement threshold.

7. The method of Claim 1, wherein the prosthetic device comprises one or more of the following: a prosthetic hand (130), a prosthetic digit, a prosthetic wrist (542C), a prosthetic arm (542D), and a prosthetic elbow (542E), and wherein the control signal comprises one or more control signals configured to cause one or more of the following: formation of a grip with the prosthetic hand (130), rotation of the prosthetic digit, rotation of the prosthetic wrist (542C), and rotation of the prosthetic elbow (542E).

8. An upper limb prosthetic control system comprising:
a prosthetic device configured to attach to a residual limb (510) of a user;
an electromyography (EMG) sensor configured to detect an EMG signal generated by a muscle contraction of the residual limb (510) of the user,
one or more motion sensors (412) configured to couple with the residual limb (510) or the prosthetic device and to detect a motion signal generated by a motion of the residual limb (510) or of the prosthetic device, wherein the one or more motion sensors (412) comprises at least one of i) an inertial measurement sensor (IMS) or ii) a magnetic field sensor (210); and
a processor in communication with the EMG sensor (104) and the one or more motion sensors (412) and configured to:
receive EMG data related to the EMG signal;
receive at least one of i) inertial measurement data related to the motion signal or ii) magnetic field data related to the motion signal;
analyze the EMG data and the at least one of the inertial measurement data or magnetic field data using a mapping matrix (418), and
generate a control signal for controlling the prosthetic device, wherein the control signal is generated in response to analyzing the EMG data and the at least one of the inertial measurement data or the magnetic field data.

9. The upper limb prosthetic control system of Claim 8, wherein the motion comprises a motion pattern, a translation, or a rotation, and wherein the muscle contraction comprises a muscle contraction pattern.

10. The upper limb prosthetic control system of Claim 8, wherein the processor is further configured to enter a control mode in response to receiving the EMG data, and then receive the at least one of the inertial measurement data or the magnetic field data.

11. The upper limb prosthetic control system of Claim 8, wherein the processor is further configured to monitor movement of the residual limb (510) to generate a movement threshold, wherein generating the control signal comprises comparison of the at least one of the inertial measurement data or the magnetic field data with the movement threshold.

12. The upper limb prosthetic control system of Claim 8, wherein the prosthetic device comprises one or more of the following: a prosthetic hand (130), a prosthetic digit, a prosthetic wrist (542C), a prosthetic arm (542D), and a prosthetic elbow (542E), and wherein the control signal comprises one or more control signals configured to cause one or more of the following: formation of a grip with the prosthetic hand (130), rotation of a prosthetic digit, rotation of the prosthetic wrist (542C), and rotation of the prosthetic elbow (542E).

13. A non-transitory computer-readable medium having instructions stored thereon that when executed by a processor performs a method of controlling an upper limb prosthetic device according to any of the claims 1 to 7, the method comprising:
receiving electromyography (EMG) data generated by an EMG sensor (104) in response to a muscle contraction of a residual limb (510) of a user of the prosthetic device,
receiving, in response to a motion of the residual limb (510) or of the prosthetic device, at least one of i) inertial measurement data generated by an inertial measurement sensor or ii) magnetic field data generated by one or more magnetic field sensors (210);
analyzing the EMG data and the at least one of the inertial measurement data or the magnetic field data using a mapping matrix (418); and
generating a control signal for controlling the prosthetic device, wherein the control signal is generated in response to analyzing the EMG data and the at least one of the inertial measurement data or the magnetic field data.

14. The non-transitory computer-readable medium of Claim 13, wherein the motion comprises a motion pattern, a translation, or a rotation, and wherein the muscle contraction comprises a muscle contraction pattern.

15. The non-transitory computer-readable medium of Claim 13, wherein the method further comprises monitoring movement of the residual limb (510) to generate a movement threshold, and wherein generating the control signal comprises comparison of the inertial measurement data with the movement threshold.

## Patentansprüche

1. Ein Verfahren zur Steuerung einer Prothese für eine obere Gliedmaße, wobei das Verfahren Folgendes umfasst:
den Empfang von Elektromyografie-Daten (EMG-Daten), die von einem EMG-Sensor (104) als Reaktion auf eine Muskelkontraktion eines Stumpfes (510) eines Benutzers der Prothese erzeugt werden,
den Empfang, als Reaktion auf eine Bewegung des Stumpfes (510) oder der Prothese mindestens eines der folgenden Elemente: i) von einem oder mehreren Trägheitsmesssensoren (IMS) generierte Trägheitsmessdaten oder ii) von einem oder mehreren Magnetfeldsensoren (210) generierte Magnetfelddaten;
die Analyse der EMG-Daten und der Trägheitsmessdaten und/oder Magnetfelddaten unter Verwendung einer Zuordnungsmatrix (418); und
das Generieren eines Steuersignals zur Steuerung der Prothese, wobei das Steuersignal in Reaktion auf die Analyse der EMG-Daten und der Trägheitsmessdaten und/oder der Magnetfelddaten generiert wird.

2. Das Verfahren nach Anspruch 1, wobei die Bewegung ein Bewegungsmuster, eine Translation oder eine Rotation umfasst.

3. Das Verfahren nach Anspruch 1, wobei die Muskelkontraktion ein Muskelkontraktionsmuster umfasst.

4. Das Verfahren nach Anspruch 1, das weiterhin den Wechsel in einen Steuermodus für die Prothese als Reaktion auf den Empfang der EMG-Daten und anschließend den Empfang der Trägheitsmessdaten und/oder der Magnetfelddaten umfasst.

5. Das Verfahren nach Anspruch 1, das weiterhin die Überwachung der Bewegung des Stumpfes (510) umfasst, um einen Bewegungsschwellenwert zu generieren, wobei das Generieren des Steuersignals den Vergleich der Trägheitsmessdaten und/oder der Magnetfelddaten mit dem Bewegungsschwellenwert umfasst.

6. Das Verfahren nach Anspruch 5, das weiterhin das Ersetzen der Bewegungsschwelle durch eine aktualisierte Bewegungsschwelle umfasst, wobei das Generieren des Steuersignals den Vergleich der Trägheitsmessdaten und/oder der Magnetfelddaten mit der aktualisierten Bewegungsschwelle umfasst.

7. Das Verfahren nach Anspruch 1, wobei die Prothese eine oder mehrere der folgenden Komponenten umfasst: eine Handprothese (130), eine Fingerprothese, eine Handgelenkprothese (542C), eine Armprothese (542D) oder eine Ellenbogenprothese (542E), und wobei das Steuersignal ein oder mehrere Steuersignale umfasst, die ausgelegt sind, um eine oder mehrere der folgenden Funktionen auszuführen: Greifen mit der Handprothese (130), Drehen der Fingerprothese, Drehen der Handgelenkprothese (542C) oder Drehen der Ellenbogenprothese (542E).

8. Ein Steuerungssystem für eine Prothese einer oberen Gliedmaße, das Folgendes umfasst:
eine Prothese, die so ausgelegt ist, dass sie an einem Stumpf (510) eines Benutzers befestigt werden kann;
einen Elektromyografie-Sensor (EMG-Sensor), der so ausgelegt ist, dass er ein EMG-Signal erfasst, das durch eine Muskelkontraktion des Stumpfes (510) des Benutzers erzeugt wird,
einen oder mehrere Bewegungssensoren (412), die so ausgelegt sind, dass sie mit dem Stumpf (510) oder der Prothese verbunden werden und ein Bewegungssignal erfassen, das durch eine Bewegung des Stumpfes (510) oder der Prothese generiert wird, wobei der eine oder die mehreren Bewegungssensoren (412) mindestens einen der folgenden Sensoren umfassen: i) einen Trägheitsmesssensor (IMS) oder ii) einen Magnetfeldsensor (210); und
einen Prozessor, der mit dem EMG-Sensor (104) und dem einen oder den mehreren Bewegungssensoren (412) in Verbindung steht und so ausgelegt ist, dass er:
EMG-Daten empfängt, die sich auf das EMG-Signal beziehen;
mindestens eines der folgenden Elemente empfängt: i) Trägheitsmessdaten, die sich auf das Bewegungssignal beziehen, oder
ii) Magnetfelddaten, die sich auf das Bewegungssignal beziehen;
die EMG-Daten und die Trägheitsmessdaten und/oder Magnetfelddaten unter Verwendung einer Zuordnungsmatrix (418) analysiert, und
ein Steuersignal zum Steuern der Prothese generiert, wobei das Steuersignal als Reaktion auf die Analyse der EMG-Daten und der Trägheitsmessdaten und/oder Magnetfelddaten generiert wird.

9. Das Steuerungssystem für eine Prothese einer oberen Gliedmaße nach Anspruch 8, wobei die Bewegung ein Bewegungsmuster, eine Translation oder eine Rotation umfasst, und wobei die Muskelkontraktion ein Muskelkontraktionsmuster umfasst.

10. Das Steuerungssystem für eine Prothese einer oberen Gliedmaße nach Anspruch 8, wobei der Prozessor weiterhin so ausgelegt ist, dass er als Reaktion auf den Empfang der EMG-Daten in einen Steuermodus wechselt und danach die Trägheitsmessdaten und/oder Magnetfelddaten empfängt.

11. Das Steuerungssystem für eine Prothese einer oberen Gliedmaße nach Anspruch 8, wobei der Prozessor weiterhin so ausgelegt ist, dass er die Bewegung des Stumpfes (510) überwacht, um einen Bewegungsschwellenwert zu generieren, wobei das Generieren des Steuersignals einen Vergleich der Trägheitsmessdaten oder/oder der Magnetfelddaten mit dem Bewegungsschwellenwert umfasst.

12. Das Steuerungssystem für eine Prothese einer oberen Gliedmaße nach Anspruch 8, wobei die Prothese eine oder mehrere der folgenden Komponenten umfasst: eine Handprothese (130), eine Fingerprothese, eine Handgelenkprothese (542C), eine Armprothese (542D) oder eine Ellenbogenprothese (542E), und wobei das Steuersignal ein oder mehrere Steuersignale umfasst, die so ausgelegt sind, dass sie eine oder mehrere der folgenden Funktionen ausführen: Greifen mit der Handprothese (130), Drehen einer Fingerprothese, Drehen der Handgelenkprothese (542C) oder Drehen der Ellenbogenprothese (542E).

13. Ein nichtflüchtiges, computerlesbares Medium mit darauf gespeicherten Anweisungen, die bei Ausführung durch einen Prozessor ein Verfahren zur Steuerung einer Prothese für eine obere Gliedmaße gemäß einem der Ansprüche 1 bis 7 ausführen, wobei das Verfahren Folgendes umfasst:
en Empfang von Elektromyografie-Daten (EMG-Daten), die von einem EMG-Sensor (104) als Reaktion auf eine Muskelkontraktion eines Stumpfes (510) eines Benutzers der Prothesenvorrichtung generiert werden,
den Empfang als Reaktion auf eine Bewegung des Stumpfes (510) oder der Prothese von i) Trägheitsmessdaten, die von einem Trägheitsmesssensor generiert werden, und/oder ii) Magnetfelddaten, die von einem oder mehreren Magnetfeldsensoren (210) generiert werden;
die Analyse der EMG-Daten und der Trägheitsmessdaten und/oder der Magnetfelddaten unter Verwendung einer Zuordnungsmatrix (418); und
das Generieren eines Steuersignals zum Steuern der Prothese, wobei das Steuersignal in Reaktion auf die Analyse der EMG-Daten und der Trägheitsmessdaten und/oder der Magnetfelddaten generiert wird.

14. Das nichtflüchtige, computerlesbare Medium nach Anspruch 13, wobei die Bewegung ein Bewegungsmuster, eine Translation oder eine Rotation umfasst und wobei die Muskelkontraktion ein Muskelkontraktionsmuster umfasst.

15. Das nichtflüchtige, computerlesbare Medium nach Anspruch 13, wobei das Verfahren weiterhin das Überwachen der Bewegung des Stumpfes (510) umfasst, um einen Bewegungsschwellenwert zu erzeugen, und wobei das Erzeugen des Steuersignals den Vergleich der Trägheitsmessdaten mit dem Bewegungsschwellenwert umfasst.

## Revendications

1. Procédé de commande d'un dispositif prothétique de membre supérieur, le procédé comprenant les étapes consistant à :
recevoir des données d'électromyographie (EMG) générées par un capteur d'EMG (104) en réponse à une contraction musculaire d'un membre résiduel (510) d'un utilisateur du dispositif prothétique,
recevoir, en réponse à un mouvement du membre résiduel (510) ou du dispositif prothétique, au moins l'une de i) données de mesure inertielle générées par un ou plusieurs capteurs de mesure inertielle (IMS) ou de ii) données de champ magnétique générées par un ou plusieurs capteurs de champ magnétique (210) ;
analyser les données d'EMG et l'au moins l'une des données de mesure inertielle ou des données de champ magnétique en utilisant une matrice de correspondance (418) ; et
générer un signal de commande afin de commander le dispositif prothétique, où le signal de commande est généré en réponse à l'analyse des données d'EMG et de l'au moins l'une des données de mesure inertielle ou des données de champ magnétique.

2. Procédé selon la revendication 1, où le mouvement comprend un schéma de mouvement, une translation, ou une rotation.

3. Procédé selon la revendication 1, où la contraction musculaire comprend un schéma de contraction musculaire.

4. Procédé selon la revendication 1, comprenant en outre le fait d'entrer un mode de commande pour le dispositif prothétique en réponse à la réception des données d'EMG, et à la réception postérieure de l'au moins l'une des données de mesure inertielle ou des données de champ magnétique.

5. Procédé selon la revendication 1, comprenant en outre le fait de surveiller un mouvement du membre résiduel (510) afin de générer un seuil de mouvement, où le fait de générer le signal de commande comprend une comparaison de l'au moins l'une des données de mesure inertielle ou des données de champ magnétique au seuil de mouvement.

6. Procédé selon la revendication 5, comprenant en outre le fait de remplacer le seuil de mouvement par un seuil de mouvement mis à jour, où le fait de générer un signal de commande comprend une comparaison de l'au moins l'une des données de mesure inertielle ou des données de champ magnétique au seuil de mouvement mis à jour.

7. Procédé selon la revendication 1, où le dispositif prothétique comprend un ou plusieurs des éléments suivants : une main prothétique (130), un doigt prothétique, un poignet prothétique (542C), un bras prothétique (542D), et un coude prothétique (542E), et où le signal de commande comprend un ou plusieurs signaux de commande configurés pour déclencher une ou plusieurs des actions suivantes : réalisation d'une préhension avec la main prothétique (130), rotation du doigt prothétique, rotation du poignet prothétique (542C), et rotation du coude prothétique (542E).

8. Système de commande prothétique de membre supérieur comprenant :
un dispositif prothétique configuré pour être attaché sur un membre résiduel (510) d'un utilisateur ;
un capteur d'électromyographie (EMG) configuré pour détecter un signal d'EMG généré par une contraction musculaire du membre résiduel (510) de l'utilisateur,
un ou plusieurs capteurs de mouvement (412) configurés pour être couplés au membre résiduel (510) ou au dispositif prothétique, où les un ou plusieurs capteurs de mouvement (412) comprennent au moins l'un i) d'un capteur de mesure inertielle (IMS) ou ii) d'un capteur de champ magnétique (210) ; et
un processeur en communication avec le capteur d'EMG (104) et les un ou plusieurs capteurs de mouvement (412) et configuré pour :
recevoir des données d'EMG associées au signal d'EMG ;
recevoir au moins l'une de i) données de mesure inertielle associées au signal de mouvement ou de ii) données de champ magnétique associées au signal de mouvement ;
analyser les données d'EMG et l'au moins l'une des données de mesure inertielle ou des données de champ magnétique en utilisant une matrice de correspondance (418) ; et
générer un signal de commande afin de commander le dispositif prothétique, où le signal de commande est généré en réponse à l'analyse des données d'EMG et de l'au moins l'une des données de mesure inertielle ou des données de champ magnétique.

9. Système de commande prothétique de membre supérieur selon la revendication 8, où le mouvement comprend un schéma de mouvement, une translation, ou une rotation, et où la contraction musculaire comprend un schéma de contraction musculaire.

10. Système de commande prothétique de membre supérieur selon la revendication 8, où le processeur est en outre configuré pour entrer un mode de commande en réponse à la réception des données d'EMG, et à la réception postérieure de l'au moins l'une des données de mesure inertielle ou des données de champ magnétique.

11. Système de commande prothétique de membre supérieur selon la revendication 8, où le processeur est en outre configuré pour surveiller un mouvement du membre résiduel (510) afin de générer un seuil de mouvement, où le fait de générer le signal de commande comprend une comparaison de l'au moins l'une des données de mesure inertielle ou des données de champ magnétique au seuil de mouvement.

12. Système de commande prothétique de membre supérieur selon la revendication 8, où le dispositif prothétique comprend un ou plusieurs des éléments suivants : une main prothétique (130), un doigt prothétique, un poignet prothétique (542C), un bras prothétique (542D), et un coude prothétique (542E), et où le signal de commande comprend un ou plusieurs signaux de commande configurés pour déclencher une ou plusieurs des actions suivantes : réalisation d'une préhension avec la main prothétique (130), rotation d'un doigt prothétique, rotation du poignet prothétique (542C), et rotation du coude prothétique (542E).

13. Support lisible par ordinateur non transitoire comprenant des instructions stockées sur celui-ci qui lorsqu'elles sont exécutées par un processeur réalise un procédé de commande d'un dispositif prothétique de membre supérieur selon l'une quelconque des revendications 1 à 7, le procédé comprenant les étapes consistant à :
recevoir des données d'électromyographie (EMG) générées par un capteur d'EMG (104) en réponse à une contraction musculaire d'un membre résiduel (510) d'un utilisateur du dispositif prothétique,
recevoir, en réponse à un mouvement du membre résiduel (510) ou du dispositif prothétique, au moins l'une de i) données de mesure inertielle générées par un capteur de mesure inertielle ou de ii) données de champ magnétique générées par un ou plusieurs capteurs de champ magnétique (210) ;
analyser les données d'EMG et l'au moins l'une des données de mesure inertielle ou des données de champ magnétique en utilisant une matrice de correspondance (418) ; et
générer un signal de commande afin de commander le dispositif prothétique, où le signal de commande est généré en réponse à l'analyse des données d'EMG et de l'au moins l'une des données de mesure inertielle ou des données de champ magnétique.

14. Support lisible par ordinateur non transitoire selon la revendication 13, où le mouvement comprend un schéma de mouvement, une translation, ou une rotation, et où la contraction musculaire comprend un schéma de contraction musculaire.

15. Support lisible par ordinateur non transitoire selon la revendication 13, où le procédé comprend en outre le fait de surveiller un mouvement du membre résiduel (510) afin de générer un seuil de mouvement, où le fait de générer le signal de commande comprend une comparaison des données de mesure inertielle au seuil de mouvement.
